(19) Europäisches Patentamt / European Patent Office / Office européen des brevets

(11) EP 1 678 505 B1

# (12) EUROPEAN PATENT SPECIFICATION

(45) Date of publication and mention of the grant of the patent:
**06.08.2014 Bulletin 2014/32**

(21) Application number: **04821180.9**

(22) Date of filing: **28.10.2004**

(51) Int Cl.:
***G01N 33/68*** *(2006.01)* ***G01N 33/50*** *(2006.01)*
***A01K 67/027*** *(2006.01)*

(86) International application number:
**PCT/EP2004/052684**

(87) International publication number:
**WO 2005/071418 (04.08.2005 Gazette 2005/31)**

## (54) DIAGNOSTIC AND THERAPEUTIC USE OF THE HUMAN DAX- 1 GENE AND PROTEIN FOR NEURODEGENERATIVE DISEASES

DIAGNOSTISCHE UND THERAPEUTISCHE VERWENDUNG DES MENSCHLICHEN DAX-1-GENS UND PROTEIN FÜR NEURODEGENERATIVE KRANKHEITEN

UTILISATION DIAGNOSTIQUE ET THERAPEUTIQUE DU GENE HUMAIN DAX-1 ET PROTEINE POUR MALADIES NEURODEGENERATIVES

(84) Designated Contracting States:
**AT BE BG CH CY CZ DE DK EE ES FI FR GB GR HU IE IT LI LU MC NL PL PT RO SE SI SK TR**

(30) Priority: **29.10.2003 US 514917 P**

(43) Date of publication of application:
**12.07.2006 Bulletin 2006/28**

(73) Proprietor: **Evotec International GmbH**
**22419 Hamburg (DE)**

(72) Inventors:
- **VON DER KAMMER, Heinz**
  **22607 Hamburg (DE)**
- **POHLNER, Johannes**
  **22175 Hamburg (DE)**
- **HESTERKAMP, Thomas**
  **22455 Hamburg (DE)**
- **EBNETH, Andreas**
  **25421 Pinneberg (DE)**

(74) Representative: **Von Kreisler Selting Werner - Partnerschaft**
**von Patentanwälten und Rechtsanwälten mbB**
**Deichmannhaus am Dom**
**Bahnhofsvorplatz 1**
**50667 Köln (DE)**

(56) References cited:
**US-A1- 2002 068 815**

- **ZANARIA ELENA ET AL: "An unusual member of the nuclear hormone receptor superfamily responsible for x-linked adrenal hypoplasia congenita" NATURE (LONDON), vol. 372, no. 6507, 1994, pages 635-641, XP002339051 ISSN: 0028-0836**
- **ECKEY MAREN ET AL: "Mixed lineage kinase 2 enhances trans-repression of Alien and nuclear receptors." MOLECULAR AND CELLULAR ENDOCRINOLOGY, vol. 213, no. 1, 31 December 2003 (2003-12-31), pages 71-78, XP002339011 ISSN: 0303-7207**
- **GUO WEIWEN ET AL: "Expression of DAX-1, the gene responsible of X-linked adrenal hypoplasia congenita and hypogonadotropic hypogonadism, in the hypothalamic-pituitary-adrenal/gonadal axis" BIOCHEMICAL AND MOLECULAR MEDICINE, vol. 56, no. 1, 1995, pages 8-13, XP002339052 ISSN: 1077-3150 cited in the application**
- **KOPP PETER: "Targeted disruption of the Ahch (Dax-1) gene: Knockout of old concepts" EUROPEAN JOURNAL OF ENDOCRINOLOGY, vol. 140, no. 4, April 1999 (1999-04), pages 291-292, XP002339200 ISSN: 0804-4643**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 678 505 B1

## Description

The present invention relates to methods of diagnosing, prognosticating and monitoring the progression of neurodegenerative diseases in a subject. Furthermore, methods of therapy control and screening for modulating agents of neurodegenerative diseases are provided. The invention also discloses pharmaceutical compositions, kits, and recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β (Aβ) protein evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). They are primarily found in the cerebral cortex and hippocampus. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge inside neurons and consist of chemically altered tau, which forms paired helical filaments twisted around each other. The appearance of neurofibrillary tangles and their increasing number correlates well with the clinical severity of AD (Schmitt et al., Neurology 2000, 55: 370-376).

AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocam pus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92). The age of onset of AD may vary within a range of 50 years, with early-onset AD occurring in people younger than 65 years of age, and late-onset of AD occurring in those older than 65 years.

Currently, there is no cure for AD, nor is there an effective treatment to halt the progression of AD or even to diagnose AD ante-mortem with high probability. Several risk factors have been identified that predispose an individual to develop AD and although there are rare examples of early-onset AD which have been attributed to genetic defects, the prevalent form of late-onset sporadic AD is of hitherto unknown etiologic origin.

The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

US-A1-2002/0068815 discloses DAX-1 protein molecules and uses thereof. The present invention is based on the differential expression of a gene coding for the DSS-AHC critical region on the X-chromosome gene 1 (DAX-1) and the protein products in human Alzheimer's disease brain samples. Various lines of evidence provide a tentative link between cholesterol metabolism and the risk and/or age of onset of development of the sporadic form of Alzheimer's disease (AD). At the genetic level, the epsilon 4 allele of the three different existing alleles (epsilon 2, 3, and 4) of the apolipoprotein E gene (ApoE) is the strongest risk factor for AD known to date (Corder et al., Science 1993, 261: 921-923; Strittmatter et al., Proc Natl Acad Sci USA 1993, 90: 1977-81; Roses, Ann NY Acad Sci 1998, 855: 738-43). As an apolipoprotein in the brain, ApoE contributes to the extracellular transport and redistribution of cholesterol and lipids in neural tissue. Modulation of the cellular content of cholesterol has been shown to directly affect the proteolytic processing of the amyloid precursor protein (APP). Specifically, a lowering of the cholesterol load favors the non-amyloidogenic α-secretase pathway, thereby precluding formation of the amylcidogenic Aβ40 and Aβ42 peptides (Howland et al., J. Biol. Chem 1998, 273: 16576-16582; Simons et al., Proc. Natl. Acad. Sci. USA 1998, 95: 6460-6464). Population-based studies have demonstrated a reduced risk of dementia following long-term treatment of individuals with inhibitors of the key enzyme of cholesterol biosynthesis, 3β-hydroxy-3β-methylglutaryl coenzyme A reductase (Jick et al., Lancet 2000, 356: 1627-1631; Wolozin et al., Arch. Neurol. 2000, 57: 1439-1443). A high dietary intake of cholesterol is considered to constitute a risk factor for dementia (Kalmijn et al., Ann. Neurol. 1997, 42: 776-782).
The regulation of enzymes involved in the metabolism of cholesterol could be relevant in the course and in the treatment of Alzheimer's disease. It is established that the steroidogenic factor 1 (SF1) plays a role in the transcriptional regulation of genes involved in cholesterol and steroid metabolism and that DSS-AHC critical region on the X-chromosome gene 1, hereinafter referred to as DAX-1, in turn acts as a negative regulator of SF1. DAX-1 has been originally identified as a gene being responsible for adrenal hypoplasia congenita, an inherited disorder of the adrenal gland development (Zanaria et al., Nature 1994, 372: 635-641) and was named DSS-AHC critical region on the X-chromosome gene 1. DSS stands for dosage-sensitive sex reversal, because patients suffering from a duplication of a segment on chromosome Xp21 develop as phenotypic females. AHC stands for adrenal hypoplasia congenita, and the disorder is characterized by the absence of the permanent zone of the adrenal cortex and a structural disorganization of the glands. In this case a deletion on the respective chromosome or a mutation of DAX-1 has been observed. The DAX-1 gene is encoded by 1413 base pairs yielding a protein of 470 amino acid in length with a molecular weight of about 51.7 kDa (protein sequence: SwissProt accession number Q96F69; mRNA sequence: Genbank accession number S74720; the DAX-1 gene nucleotide sequence and the corresponding DAX-1 protein amino acid sequence is disclosed in US patent 6465627 by McCabe et al.). The gene consists of two exons which are separated by an intron spanning 3.4 kb (Guo et al., J. Endocrinol. Met. 1996, 81: 2481-2486). The predicted amino acid sequence of the C-terminal region of DAX-1 reveals similarities to the nuclear hormone receptor gene family (domain E) which is involved in ligand binding (e.g. retinoic acid receptor). The N-terminal half, which may be divided into three and a half repeats of 65 to 67-amino acids each, has been speculated to consist of a novel unusual zinc-finger DNA-binding motif (Burris et al., Biochem. Biophys. Res. Comm. 1995, 214: 576-581; Guo et al., J. Clin. Endocrinol. Met. 1996, 81: 2481-2486).
DAX-1 is expressed predominantly in the adrenal gland and gonads (Zanaria et al., Nature 1994, 372: 635-641) but has also been found in human skin (Patel et al., J. Invest. Dermatol. 2001, 117: 1559-1565), hypothalamus and pituitary (Guo et al., Biochem. Mol. Med. 1995, 56: 8-13). Intracellularly, DAX-1 may be found in the nucleus as well as in the cytoplasm, and it has been speculated that DAX-1 might function as a shuttling RNA binding protein which is associated with polyribosomes via mRNA (Lalli et al., Mol. Cell Biol. 2000, 20: 4910-4921). Mutations of DAX-1 as found in X-linked adrenal hypoplasia congenita cause a shift in the localization of the protein to the cytoplasm although the N-terminal nuclear localization signal is intact (Lehmann et al., Proc. Natl. Acad. Sci. USA 2002, 99: 8225-8230). As DAX-1 is expressed mainly in tissues involved in steroidogenesis, a role in the transcriptional regulation of genes participated in the metabolism of steroids has been proposed. The function of DAX-1 may be best described as a transcriptional repressor. indeed, it has been shown that DNA-binding of DAX-1 results in transcriptional repression of certain genes, leading to a drastic decrease of steroid production (Zazopoulos et al., Nature 1997, 390: 311-315). The expression pattern of DAX-1 thereby overlaps with the expression of the steroidogenic factor 1 (SF-1), which is well known to function as a transcription factor for a variety of enzymes involved in steroidogenesis. A balance between repressor and inducer function of DAX-1 and SF-1 seems to be of critical importance in the regulation of steroid metabolism (Osman et al., J. Biol. Chem. 2002, 277: 41259-41267). However, DAX-1 may also be present in cells that do not express SF-1, suggesting a role in other pathways besides those regulated by SF-1 (Ikeda et al., Dev. Dyn. 2001, 220: 363-376).

The transcription of several genes involved in steroidogenesis is repressed by DAX-1 (Lalli et al., Mol. Endocrinol. 2003, 17: 1445-1453), among them being StAR (steroidogenic acute regulatory protein; Zazopoulos et al., Nature 1997, 390: 311-315; Lalli et al., Mol. Endocrinol. 1997, 11: 1950-1960), P450scc (cytochrome P450 side chain cleavage enzyme), 3beta-HSD (3beta-hydroxy-delta 5-C27-steroid oxidoreductase; Lalli et al., Endocrinol. 1998, 139: 4237-4243), CYP17 (steroid 17-alpha-hydroxylase, Hanley et al., Mol. Endocrinol. 2001, 15: 57-68) and CYP19 (aromatase, Wang et al., Proc. Nat. Acad. Sci. USA 2001, 98: 7988-7993). It is speculated that DAX-1 exerts its function by binding to a hairpin structure in the promoter of the respective genes where it might allosterically inhibit the binding of the steroidogenic factor 1, which has been described to drive the expression of the genes (Zazopoulos et al., Nature 1997, 390: 311-315). The expression of DAX-1 has been speculated to be regulated by the steroidogenic factor 1. A consensus site for SF-1 has been found in the promoter region of DAX-1 (Burris et al., Biochem. Biophys. Res. Comm. 1995, 214: 576-581). However, DAX-1 expression in SF-1-knock out mice was unimpaired suggesting that SF-1 might not be required to drive DAX-1 expression (Ikeda et al., Mol. Endocrinol. 1996, 10: 1261-1272).

In a review from Kopp Peter the phenotypes of transgenic overexpression and targeted disruption of the mouse homologue Ahch is described (European Journal of Endocrinology 1999, 140: 291-292). The studies suggest that Ahch is essential for normal development and function of the testis.

To test whether various DAX-1 levels may contribute to cholesterol homeostasis in the physiological or pathophysiological human brain, transcription levels of the DAX-1 gene were measured by RT-PCR in the frontal and temporal cortices and in the hippocampus of AD patients and age-matched healthy controls.
The instant invention discloses a dysregulation of DAX-1 gene expression in Alzheimer's disease-affected brains, in that DAX-1 mRNA levels are higher in the temporal cortex and in the hippocampus as compared to the frontal cortex of AD patients, and in that DAX-1 mRNA levels are elevated in the temporal cortex but not frontal cortex of AD-patients compared to controls. DAX-1 expression does not differ between the temporal and frontal cortex and between the hippocampus and frontal cortex of healthy age-matched control subjects. This dysregulation may relate to a pathologic alteration of cholesterol homeostasis in AD-affected brains. For instance, such dysregulation could cause a pro-apoptotic imbalance favoring cell death in the affected brain regions, eventually leading to irreversible neuronal damage. To date, no experiments have been described that demonstrate a relationship between the dysregulation of DAX-1 gene expression and the pathology of neurodegenerative diseases, in particular AD. Likewise, no mutations in the DAX-1 gene have been decribed to be associated with said diseases. Linking the DAX-1 gene to such diseases offers new ways, inter alia, for the diagnosis and treatment of said diseases.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity or to biological activity and/or pharmacological activity which refers to binding, antagonization, repression, blocking or neutralization. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For the purpose of clarity, a derivative transcript, for instance, refers to a transcript having alterations in the nucleic acid sequence such as single or multiple nucleotide deletions, insertions, or exchanges. A "derivative" may be generated by processes such as altered phosphorylation, or glycosylation, or acetylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in the biological activity and/or pharmacological activity, in enzyme activity, a change in binding characteristics, or any other change or alteration in the biological, functional, or immunological properties of said translation product of a gene. A "modulator" refers to a molecule which has the capacity to either enhance or inhibit, thus to "modulate" a functional property of an ion channel subunit or an ion channel, to "modulate" binding, antagonization, repression, blocking, neutralization or sequestration of an ion channel or ion channel subunit and to "modulate" activation, agonization and upregulation. "Modulation" will be also used to refer to the capacity to affect the biological activity of a cell. The terms "modulator", "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. They may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. Such modulators, agents, reagents or compounds can be factors present in cell culture media, or sera used for cell culturing, factors such as trophic factors. "Trophic factors" as used in the present invention include but are not limited to neurotrophic factors, to neuregulins, to cytokines, to neurokines, to neuroimmune factors, to factors derived from the brain (BDNF) and to factors of the TGF beta family. Examples of such trophic factors are neurotrophin 3 (NT-3), neurotrophin 4/5 (NT-4/5), nerve growth factor (NGF), fibroblast growth factor (FGF), epidermal growth factor (EGF), interleukin-beta, glial cell-derived neurotrophic factors (GDNF), ciliary neurotrophic factor (CNTF), insulin-like growth factor (IGF), transforming growth factor (TGF) and platelet-derived growth factor (PDGF). The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. In the art, the terms "identity" and "similarity" mean the degree of polypeptide or polynucleotide sequence relatedness which are determined by matching a query sequence and other sequences of preferably the same type (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Biol. 1990, 215: 403-410; Altschul et al., Nucleic Acids Res. 1997, 25: 3389-3402; Devereux et al., Nucleic Acids Res. 1984, 12: 387), BLASTN 2.0 (Gish W., http://blast.wusti.edu, 1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; Needleman and Wunsch, J. Mol. Biol. 1970, 48: 443-453). The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of DAX-1, of SEQ ID NO. 1. "Variants" of a protein molecule include, for example, proteins with conservative amino acid substitutions in highly conservative regions. "Proteins and polypeptides" of the present invention include variants, fragments and chemical derivatives of the protein comprising the amino acid sequences of DAX-1, of SEQ ID NO. 1. Sequence variations shall be included wherein a codon are replaced with another codon due to alternative base sequences, but the amino acid sequence translated by the DNA sequence remains unchanged. This known in the art phenomenon is called redundancy of the set of codons which translate specific amino acids. Included shall be such exchange of amino acids which would have no effect on functionality, such as arginine for lysine, valine for leucine, asparagine for glutamine. Proteins and polypeptides can be included which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules or substances which have been changed and/or that are removed from their natural environment, i.e. isolated from a cell or from a living organism in which they normally occur, and that are separated or essentially purified from the coexisting components with which they are found to be associated in nature, it is also said that they are "non-native". This notion further means that the sequences encoding such molecules can be linked by the hand of man to polynucleotides to which they are not linked in their natural state and such molecules can be produced by recombinant and/or synthetic means (non-native). Even if for said purposes those sequences may be introduced into living or non-living organisms by methods known to those skilled in the art, and even if those sequences are still present in said organisms, they are still considered to be isolated, to be non-native. In the present invention, the terms "risk", "susceptibility", and "predisposition" are tantamount and are used with respect to the probability of developing a neurodegenerative disease, preferably Alzheimer's disease.

The term "AD" shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998). The term "Braak stage" or "Braak staging" refers to the classification of brains according to the criteria proposed by Braak and Braak (Braak and Braak, Acta Neuropathology 1991, 82: 239-259). On the basis of the distribution of neurofibrillary tangles and neuropil threads, the neuropathologic progression of AD is divided into six stages (stage 0 to 6). In the instant invention Braak stages 0 to 2 represent healthy control persons ("controls"), and Braak stages 4 to 6 represent persons suffering from Alzheimer's disease ("AD patients"). The values obtained from said "controls" are the "reference values" representing a "known health status" and the values obtained from said "AD patients" are the "reference values" representing a "known disease status". Braak stage 3 may represent either a healthy control persons or an AD patient. The higher the Braak stage the more likely is the possibility to display the symptoms of AD. For a neuropathological assessment, i.e. an estimation of the probability that pathological changes of AD are the underlying cause of dementia, a recommendation is given by Braak H. (www. alzforum.org).

Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Conditions involving neurodegenerative processes are, for instance, age-related macular degeneration, narcolepsy, motor neuron diseases, prion diseases and traumatic nerve injury and repair, and multiple sclerosis.

In one aspect, the invention features a method of diagnosing or prognosticating a neurodegenerative disease in a subject, or determining whether a subject is at increased risk of developing said disease. The method comprises: determining a level, or an activity, or both said level and said activity of (i) a transcription product of a gene coding for DAX-1, and/or of (ii) a translation product of a gene coding for DAX-1 in a sample from said subject and comparing said level, and/or said activity to a reference value representing a known disease or health status, thereby diagnosing or prognosticating said neurodegenerative disease in said subject, or determining whether said subject is at increased risk of developing said neurodegenerative disease. The wording "in a subject" refers to results of the methods disclosed as far as they relate to a disease afflicting a subject, that is to say, said disease being "in" a subject.

The invention also discloses the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further discloses the detection and the production of said nucleic acid sequences, or fragments and/or variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit. Primers for DAX-1 are exemplarily described in Example (iii).

In a further aspect, the invention features a method of monitoring the progression of a neurodegenerative disease in a subject. A level, or an activity, or both said level and said activity, of (i) a transcription product of a gene coding for DAX-1, and/or of (ii) a translation product of a gene coding for DAX-1 in a sample from said subject is determined. Said level and/or said activity is compared to a reference value representing a known disease or health status. Thereby the progression of said neurodegenerative disease, of Alzheimer's disease, in said subject is monitored.

In still a further aspect, the invention discloses a method of evaluating a treatment for a neurodegenerative disease, comprising determining a level, or an activity, or both said level and said activity of (i) a transcription product of a gene coding for DAX-1, and/or of (ii) a translation product of a gene coding for DAX-1 in a sample obtained from a subject being treated for said disease. Said level, or said activity, or both said level and said activity are compared to a reference value representing a known disease or health status, thereby evaluating the treatment for said neurodegenerative disease, for Alzheimer's disease.

In a preferred embodiment of the herein claimed methods, kits, recombinant animals, molecules, assays, and uses of the instant invention, said DAX-1 gene, also referred to as DSS-AHC critical region on the X-chromosome gene 1, or termed NR0B1, AHCH or AHX, is represented by the sequence of SEQ ID NO. 1 (Genbank accession number Q96F69, which is deduced from the mRNA corresponding to the cDNA sequence of Genbank accession number S74720), SEQ ID NO. 2 and SEQ ID NO. 3 which corresponds to the coding sequence of DAX-1 (DAX-1 cds). In the instant invention, the gene coding for said DAX-1 protein is also generally referred to as the DAX-1 gene, or simply DAX-1. Further, the protein of DAX-1 encoded by the DAX-1 gene is also generally referred to as the DAX-1 protein, or simply DAX-1. Said sequences are "isolated" as the term is employed herein.

In a further preferred embodiment of the herein claimed methods, recombinant animals, molecules, assays, and uses of the instant invention, said neurodegenerative disease or disorder is Alzheimer's disease, and said subjects or patients suffer from Alzheimer's disease.

The present invention discloses the differential expression, the differential regulation, a dysregulation of a gene coding for DAX-1 in specific samples, in specific brain regions of AD patients and/or in comparison to control persons. Further, the present invention discloses that the gene expression of DAX-1 is varied, is dysregulated in AD-affected brains, in that DAX-1 mRNA levels are up-regulated or elevated in the temporal cortex and/or the hippocampus as compared to the frontal cortex or are down-regulated in the frontal cortex as compared to the temporal cortex and/or the hippocampus. Further, the present invention discloses that the DAX-1 expression differs between the frontal cortex and the temporal cortex and/or the hippocampus of healthy age-matched control subjects compared to the frontal cortex and the temporal cortex and/or the hippocampus of AD patients. No such dysregulation is observed in samples obtained from age-matched, healthy controls. To date, no experiments have been described that demonstrate a relationship between the dysregulation of DAX-1 gene expression and the pathology of neurodegenerative disorders, in particular AD. The link of the DAX-1 gene and the encoded DAX-1 proteins to such diseases, as disclosed in the present invention, offers new ways, inter alia, for the diagnosis and treatment of said disorders, in particular AD.

Neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions and display a selective vulnerability to neuronal loss and degeneration in AD. In contrast, neurons within the frontal cortex, the occipital cortex, and the cerebellum remain largely intact and preserved from neurodegenerative processes. Brain tissues from the frontal cortex (F), the temporal cortex (T), and the hippocampus (H) of AD patients and healthy, age-matched control individuals were used for the herein disclosed examples. Consequently, the DAX-1 gene and its corresponding transcription and/or translation products have a causative role in the regional selective neuronal degeneration typically observed in AD. DAX-1 confers a neuroprotective function to the remaining surviving nerve cells as disclosed and described in the instant invention. Based on these disclosures, the present invention has utility for the diagnostic evaluation and prognosis as well as for the identification of a predisposition to a neurodegenerative disease, in particular AD. Furthermore, the present invention provides methods for the diagnostic monitoring of patients undergoing treatment for such a disease.

It is preferred that said sample to be analyzed and determined is selected from the group comprising brain tissue, brain cells or other tissues or other body cells. The sample can also comprise cerebrospinal fluid or other body fluids including saliva, urine, blood, serum plasma, or mucus. Preferably, the methods of diagnosis, prognosis, monitoring the progression or evaluating a treatment for a neurodegenerative disease, according to the instant invention, can be practiced *ex corpore,* and such methods preferably relate to samples, for instance, body fluids or cells, removed, collected, or isolated from a subject or patient or healthy control person.

In further preferred embodiments, said reference value is that of a level, for an activity, or both said level and said activity of (i) a transcription product of a gene coding for DAX-1, and/or of (ii) a translation product of a gene coding for DAX-1 in a sample obtained from a subject not suffering from said neurodegenerative disease (healthy control person, control sample, control) or in a sample obtained from a subject suffering from a neurodegenerative disease, in particular Alzheimer's disease (patient sample, patient).

In preferred embodiments, an alteration in the level and/or activity, a varied level and/or activity of a transcription product of the gene coding for DAX-1 and/or of a translation product of the gene coding for DAX-1 and/or of a fragment, or derivative, or variant thereof, in a sample cell, or tissue, or body fluid obtained from a subject relative to a reference value representing a known health status (control sample) indicates a diagnosis, or prognosis, or increased risk of becoming diseased with a neurodegenerative disease, particularly AD.

In further preferred embodiments, an equal or similar level and/or activity of a transcription product of the gene coding for a DAX-1 protein and/or of a translation product of the gene coding for a DAX-1 protein in a sample cell, or tissue, or body fluid obtained from a subject relative to a reference value representing a known disease status of a neurodegenerative disease, in particular Alzheimer's disease (AD patient sample), indicates a diagnosis, or prognosis, or increased risk of becoming diseased with said neurodegenerative disease.

In preferred embodiments, measurement of the level of transcription products of a gene coding for DAX-1 is performed in a sample obtained from a subject using a quantitative PCR-analysis with primer combinations to amplify said gene specific sequences from cDNA obtained by reverse transcription of RNA extracted from a sample of a subject. Primer combinations are given in Example (iii) of the instant invention, but also other primers generated from the sequences as disclosed in the instant invention can be used. A Northern blot with probes specific for said gene can also be applied. It might further be preferred to measure transcription products by means of chip-based micro-array technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of a gene coding for DAX-1 and/or a level of activity of said translation product, can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Using Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip basted technologies (see Schena M., Microarray Biochip Technology, Eaton Publishing, Natick, MA, 2000).

In a preferred embodiment, the level, or the activity, or both said level and said activity of (i) a transcription product of a gene coding for DAX-1, and/or of (ii) a translation product of a gene coding for DAX-1, in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect, the invention discloses a kit for diagnosing or prognosticating neurodegenerative diseases, in particular AD, in a subject, or determining the propensity or predisposition of a subject to develop a neurodegenerative disease, in particular AD, said kit comprising:

(a) at least one reagent which is selected from the group consisting of (i) reagents that selectively detect a transcription product of a gene coding for DAX-1 (ii) reagents that selectively detect a translation product of a gene coding for DAX-1;
and

(b) instructions for diagnosing, or prognosticating a neurodegenerative disease, in particular AD, and/or determining the propensity or predisposition of a subject to develop such a disease by describing the steps of:

- detecting a level, or an activity, or both said level and said activity, of said transcription product and/or said translation product of a gene coding for DAX-1, in a sample obtained from said subject; and
- diagnosing or prognosticating a neurodegenerative disease, in particular AD and/or determining the propensity or predisposition of said subject to develop such a disease, wherein a varied or altered level, or activity, or both said level and said activity, of said transcription product and/or said translation product compared to a reference value representing a known health status (control) and/or wherein a level, or activity, or both said level and said activity, of said transcription product and/or said translation product is similar or equal to a reference value representing a known disease status, preferably a disease status of AD, indicates a diagnosis or prognosis of a neurodegenerative disease, in particular AD, or an increased propensity or predisposition of developing such a disease. The kit, disclosed by the present invention, may be particularly useful for the identification of individuals that are at risk of developing a neurodegenerative disease, in particular AD.

In a further aspect the invention discloses the use of a kit in a method of diagnosing or prognosticating a neurodegenerative disease, in particular Alzheimer's disease, in a subject, and in a method of determining the propensity or predisposition of a subject to develop such a disease by the steps of: (i) detecting in a sample obtained from said subject a level, or an activity, or both said level and said activity of a transcription product and/or of a translation product of a gene coding for DAX-1, and (ii) comparing said level or activity, or both said level and said activity of a transcription product and/or of a translation product of a gene coding for DAX-1 to a reference value representing a known health status and/or to a reference value representing a known disease status, and said level, or activity, or both said level and said activity, of said transcription product and/or said translation product is varied compared to a reference value representing a known health status, and/or is similar or equal to a reference value representing a known disease status.
Consequently, the kit, according to the present invention, may serve as a means for targeting identified individuals for early preventive measures or therapeutic intervention prior to disease onset, before irreversible damage in the course of the disease has been inflicted. Furthermore, in preferred embodiments, the kit featured in the invention is useful for monitoring a progression of a neurodegenerative disease, in particular AD in a subject, as well as monitoring and evaluating success or failure of a therapeutic treatment for such a disease of said subject.

In another aspect, the invention discloses a method of treating or preventing a neurodegenerative disease, in particular AD, in a subject comprising the administration to said subject in a therapeutically or prophylactically effective amount of an agent or agents which directly or indirectly affect a level, or an activity, or both said level and said activity, of (i) a gene coding for DAX-1, and/or (ii) a transcription product of a gene coding for DAX-1, and/or (iii) a translation product of a gene coding for DAX-1, and/or (iv) a fragment, or derivative, or variant of (i) to (iii). Said agent may comprise a small molecule, or it may also comprise a peptide, an oligopeptide, or a polypeptide. Said peptide, oligopeptide, or polypeptide may comprise an amino acid sequence of a translation product of a gene coding for DAX-1, or a fragment, or derivative, or a variant thereof. An agent for treating or preventing a neurodegenerative disease, in particular AD, according to the instant invention, may also consist of a nucleotide, an oligonucleotide, or a polynucleotide. Said oligonucleotide or polynucleotide may comprise a nucleotide sequence of the gene coding for DAX-1, either in sense orientation or in antisense orientation.

In preferred embodiments, the method comprises the application of per se known methods of gene therapy and/or antisense nucleic acid technology to administer said agent or agents. In general, gene therapy includes several approaches: molecular replacement of a mutated gene, addition of a new gene presuming in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

In particular, the invention discloses a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechnol 1995, 13: 197-199; Crooke, Biotechnology 1992, 10: 882-6). Apart from hybridization strategies, the application of ribozym es, i.e. RNA molecules that act as enzymes, destroying RNA that carries the message of disease has also been described (see e.g. Barinaga, Science 1993, 262: 1512-1514). Preferably the subject to be treated is a human, and therapeutic antisense nucleic acids or derivatives thereof are directed against transcripts of a gene coding for DAX-1. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligo-deoxynucleotides are known to those of skill in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA interference (RNAi), can be another effective approach for nucleic acid therapy (Hannon, Nature 2002, 418: 244-251).

In further preferred embodiments, the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection (see Mc Celland and Pardee, Expression Genetics: Accelerated and High-Throughput Methods, Eaton Publishing, Natick, MA, 1999).

In preferred embodiments, said agent for treating and preventing a neurodegenerative disease, in particular AD, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

In a further aspect, the invention features a modulator of an activity, or a level, or both said activity and said level of at least one substance which is selected from the group consisting of (i) a gene coding for DAX-1, and/or (ii) a transcription product of a gene coding for DAX-1, and/or (iii) a translation product of a gene coding for DAX-1.

In an additional aspect, the invention discloses a pharmaceutical composition comprising said modulator and preferably a pharmaceutical carrier. Said carrier refers to a diluent, adjuvant, excipient, or vehicle with which the modulator is administered.

In a further aspect, the invention features a modulator of an activity, or a level, or both said activity and said level of at least one substance which is selected from the group consisting of (i) a gene coding for DAX-1, and/or (ii) a transcription product of a gene coding for DAX-1, and/or (iii) a translation product of a gene coding for DAX-1 for use in a pharmaceutical composition.

In another aspect, the invention provides for the use of a modulator of an activity, or a level, or both said activity and said level of at least one substance which is selected from the group consisting of (i) a gene coding for DAX-1 and/or (ii) a transcription product of a gene coding for DAX-1 and/or (iii) a translation product of a gene coding for DAX-1 for a preparation of a medicament for treating or preventing a neurodegenerative disease, in particular AD.

In one aspect, the present invention also provides a kit comprising one or more containers filled with a therapeutically or prophylactically effective amount of said pharmaceutical composition.

In another aspect, the present invention discloses the use of non-native nucleic acid molecules and of translation products, protein molecules of the gene coding for human and/or mouse DAX-1 (DSS-AHC critical region on the X-chromosome gene 1) and/or fragments, or derivatives, or variants thereof, of nucleic acid molecules as shown in SEQ ID NO. 2, SEQ ID NO. 3 and protein molecules as shown in SEQ ID NO. 1, as targeting molecules to generate recombinant, genetically altered non-human animals which are transgenic animals and/or knockout animals. It is preferred that said genetically altered non-human animal is a mammal, preferably a rodent, more preferably a mouse or a rat or a guinea pig. It is further preferred that said genetically altered non-human animal is an invertebrate animal, preferably an insect, more preferably a fly such as the fly *Drosophila melanogaster.* Further, said genetically altered non-human animal may be a domestic animal, or a non-human primate. In one embodiment, the expression of said genetic alteration results in a non-human animal exhibiting a predisposition to developing symptoms and/or displaying symptoms of neuropathology similar to a neurodegenerative disease, in particular symptoms of a neuropathology similar to AD (AD-type neuropathology), including, inter alia , histological features of AD and behavioural changes characteristic of AD. In another embodiment, the expression of said genetic alteration results in a non-human animal which has a reduced risk of developing symptoms similar to a neurodegenerative disease, in particular a reduced risk of developing symptoms of a neuropathology similar to AD and/or which shows a reduction of AD symptoms and/or which has no AD symptoms due to a beneficial effect caused by the expression of the gene used to genetically alter said non-human animal.

In one aspect, the invention features a recombinant, genetically altered non-human animal comprising a non-native gene sequence coding for DAX-1 (DSS-AHC critical region on the X-chromosome gene 1), as shown in SEQ ID NO. 2, SEQ ID NO. 3 and as shown in SEQ ID NO. 1. Said non-native gene sequence coding for DAX-1 may be either the human and/or the mouse DAX-1 gene sequence. The generation of said recombinant, genetically altered non-human animal comprises (i) the use of non-native nucleic acid molecules and of translation products, protein molecules of the gene coding for human and/or mouse DAX-1, as shown in SEQ ID NO. 2, SEQ ID NO. 3, SEQ ID NO. 1 for generating a gene targeting construct and (ii) providing said gene targeting construct containing a gene sequence of human and/or mouse DAX-1, and a selectable marker sequence, and (iii) introducing said targeting construct into a stem cell, into an embryonal stem (ES) cell of a non-human animal, and (iv) introducing said non-human animal stem cell into a non-human embryo, and (v) transplanting said embryo into a pseudopregnant non-human animal, and (vi) allowing said embryo to develop to term, and (vii) identifying a genetically altered non-human animal whose genome comprises a modification of said gene sequence in one or both alleles, and (viii) breeding the genetically altered non-human animal of step (vii) to obtain a genetically altered non-human animal whose genome comprises a modification of said endogenous gene. It is preferred that said genetically altered non-human animal expresses a recombinant, an altered gene wherein said expression is a mis-expression, or under-expression, or over-expression, or non-expression. Examples of such targeting constructs containing a gene sequence of human and/or mouse DAX-1 and a selectable marker sequence, as well as the expression of said recombinant, altered DAX-1 genes in non-human genetically altered animals, preferably animals such as mouse or fly, are disclosed in the present invention (see Examples (Viii), (ix) and Figures 14 to 20).
In one preferred embodiment, said gene disruption or suppression or activation or the expression of said genetic alteration results in said non-human animal exhibiting a predisposition to developing symptoms, and/or displaying symptoms of neuropathology similar to a neurodegenerative disease, in particular symptoms of a neuropathology similar to AD (AD-type neuropathology).

In another preferred embodiment, the expression of said genetic alteration results in a non-human animal which has a reduced risk of developing symptoms similar to a neurodegenerative disease, in particular a reduced risk of developing symptoms similar to AD and/or which shows a reduction of AD symptoms and/or which has no AD symptoms due to an effect, which can be a beneficial effect, caused by the expression of the gene used to genetically alter said non-human animal.

In a further preferred embodiment of the present invention, said genetically altered non-human animal is a mammal, preferably a rodent, more preferably a mouse or a rat or a guinea pig. It is further preferred that said genetically altered non-human animal is an invertebrate animal, preferably an insect, more preferably a fly such as the fly *Drosophila melanogaster*. Further, said genetically altered non-human animal may be a domestic animal, or a non-human primate. Said genetically altered non-human is a transgenic animal and/or a knockout animal.

In a further preferred embodiment of the present invention, said recombinant, genetically altered non-human animal whose genome comprises a non-native gene sequence coding for either the human and/or the mouse DAX-1, which is generated by the steps of (i) - (viii) and as described in the present invention, is crossed to an Alzheimer's disease animal model as commonly known in the art to produce a transgenic DAX-1 animal and/or DAX-1 knock-out animal on an Alzheimer's disease background. The impact of DAX-1 expression on Alzheimer's disease pathology in said genetically altered non-human transgenic DAX-1 animal and/or DAX-1 knock-out animal is defined by histological analyses, immunohistochemistry and/or quantification of diffuse and mature plaques in the brain, by staining for certain cell populations and/or for signs of inflammation and neurodegeneration and further, by biochemical analyses like differential extraction of Abeta and/or phosphorylation status of Tau protein. The neurological function is assessed by a battery of behavioural tests including but are not limited to minineurological examinations, rotarod, grip test, hotplate test, zero maze, openfield test, Y maze, Morris water maze and/or active avoidance test. Further, the phenotype of said non-human transgenic DAX-1 animal and/or DAX-1 knock-out animal is analyzed using gene expression analyses, protein detection methods and histopathology of a variety of organs.

In further preferred embodiment Alzheimer's disease animal models which are used for the crossing with transgenic DAX-1 animals and/or DAX-1 knock-out animals are selected from genetically altered mice and/or flies expressing human Alzheimer Precursor Protein (APP) and/or mutant forms of APP, e.g. APP with the swedish mutation, and/or human Presenilin-1 or -2 with known mutations as described in the literature (Janus and Westaway, Physiology Behavior 2001, 873-886; Richards et al., J. Neuroscience 2003, 23:8989-9003) and/or human Tau with known mutations, e.g. the P301 L mutation (Götz et al., J. Biological Chemistry 2001, 276:529-534) or double or triple transgenic animals from those or other mouse mutants developing Alzheimer-like pathologies. Further, genetically altered non-human animals are selected such as human APP (hAPP) and *Drosophila* Presenilin transgenic flies, as for example the UAS-APP695II and the UAS-DPsn-mutants (L235P), such as UAS-bovine TAU transgenic flies, actin-GAL4 flies and/or gmr-GAL4 flies. Other Alzheimer's disease animal models can be recombinant animal models which are capable of producing neurofibrillary tangles and/or amyloid plaques; recombinant animal models which express a recombinant gene coding for a tau protein, such as human or mouse tau or tau isoforms as the four-repeat isoform or the P301 L mutant tau; recombinant animal models which express a recombinant gene coding for an amyloid precursor protein or a mutant amyloid precursor protein, or beta-amyloid; recombinant animal models which express a recombinant gene coding for a secretase, gamma-secretase, beta-secretase or alpha-secretase, Presenilin1 or Presenilin2; and any recombinant animal models which express a combination of the recombinant genes as described above.

In preferred embodiment of the present invention, said crossing results in DAX-1 knockout animals and/or transgenic DAX-1 animals on an Alzheimer's disease background which feature a strengthened and boosted predisposition to develop symptoms and/or to display symptoms of neuropathology similar to a neurodegenerative disease, in particular symptoms of a neuropathology similar to AD (AD-type neuropathology), including inter alia histological features of AD and behavioural changes characteristic of AD.

In another preferred embodiment of the present invention, said crossing results in DAX-1 knockout animals and/or transgenic DAX-1 animals on an Alzheimer's disease background which have a reduction of AD symptoms and/or a reduced risk of developing symptoms similar to a neurodegenerative disease, in particular a reduced risk of developing symptoms of a neuropathology similar to AD, or showing no AD symptoms due to a beneficial effect caused by the expression of the gene used to genetically alter said non-human animal.

The genetically altered non-human transgenic animal and/or a knockout animal can be used as an experimental animal, as a test animal, as an animal model for a neurodegenerative disorder, preferably as an animal model for Alzheimer. Examples of such genetically altered non-human animals showing such neuropathological features and/or showing reduced symptoms are disclosed in the present invention (see Examples (Viii), (ix) and Figures 14 to 20). Strategies and techniques for the generation and construction of such a transgenic and/or knockout animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999) and are described in detail in the present invention (see above and Examples (Viii), (ix) and Figures 14 to 20).

In a further aspect of the present invention, it is preferred to make use of such a recombinant, genetically altered non-human animal, transgenic or knockout animal, as an animal model for investigating neurodegenerative diseases, in particular Alzheimer's disease. Such an animal may be a test animal or an experimental animal useful for screening, testing and validating compounds, agents and modulators in the development of diagnostics and therapeutics to treat neurodegenerative diseases, in particular Alzheimer's disease.

In one further aspect, the invention features a screening assay for a modulator of neurodegenerative diseases, in particular AD, or related diseases and disorders of one or more substances selected from the group consisting of (i) a gene coding for DAX-1, and/or (ii) a transcription product of a gene coding for DAx-1, and/or (iii) a translation product of a gene coding for DAx-1, and/or (iv) a fragment, or derivative, or variant of (i) to (iii), comprising (a) administering a test compound to a test animal or experimental animal or animal model, which is predisposed to developing or has already developed symptoms of a neurodegenerative disease or related diseases or disorders, and (b) measuring the activity and/or level of one or more substances recited in (i) to (iv), and (c) measuring the activity and/or level of said substances in a matched control animal which is equally predisposed to developing or has already developed symptoms of said diseases and to which animal no such test compound has been administered, and (d) comparing the activity and/or level of the substance in the animals of step (b) and (c), wherein an alteration in the activity and/or level of substances in the test animal, or experimental animal, or animal model indicates that the test compound is a modulator of said diseases and disorders.

In a preferred embodiment, said test animal, or experimental animal, or animal model and/or said control animal is a recombinant, genetically altered non-human animal which expresses a gene coding for DAX-1, under the control of a transcriptional regulatory element which is not the native DAX-1 gene transcriptional control regulatory element, as disclosed in the present invention (see below).
In a further aspect, the genetically altered non-human animals according to the present invention provide an in-vivo assay to determine or validate the efficacy of therapies, or modulatory agents, or compounds for the treatment of neurodegenerative diseases, in particular Alzheimer's disease.

In another aspect, the invention features an assay for screening for a modulator, or an agent, or compound of neurodegenerative diseases, in particular AD, or related diseases and disorders of one or more substances selected from the group consisting of (i) a gene coding for DAX-1, and/or (ii) a transcription product of a gene coding for DAX-1, and/or (iii) a translation product of a gene coding for DAX-1. This screening method comprises (a) contacting a cell with a test compound, agent, or modulator and (b) measuring the activity, or the level, or both the activity and the level of one or more substances recited in (i) to (iv), and (c) measuring the activity, or the level, or both the activity and the level of said substances in a control cell not contacted with said test compound, and (d) comparing the levels of the substance in the cells of step (b) and (c), wherein an alteration in the activity and/or level of said substances in the contacted cells, or the contacted cells, indicates that the test compound, or agent, or modulator, is a modulator of said diseases and disorders, wherein said modulator can be the activity, or the level, or both the activity and the level of one or more substances recited in (i) to (iii).
Examples of cells used in said screening assay, such as cells over-expressing the DAX-1 protein, preferably stably over-expressing the DAX-1 protein, as disclosed in the present invention, are given below (Example (vi) and Figure 12). A detailed example of an assay, using said cells is disclosed in the present invention (see Example (vii) and Figure 13).
The examples of the genetically altered animals and cells and screening assays as disclosed, are illustrative only and not intended to limit the remainder of the disclosure in any way.

In another embodiment, the present invention provides a method for producing a medicament comprising the steps of (i) identifying a modulator of neurodegenerative diseases by a method of the aforementioned screening assays and (ii) admixing the modulator with a pharmaceutical carrier. However, said modulator may also be identifiable by other types of screening assays.

In another aspect, the present invention provides for an assay for testing a compound, preferably for screening a plurality of compounds, for inhibition of binding between a ligand and DAX-1 protein, or a fragment, or derivative, or variant thereof. Said screening assay comprises the steps of (i) adding a liquid suspension of said DAX-1 protein, or a fragment, or derivative, or variant thereof, to a plurality of containers, and (ii) adding a compound or a plurality of compounds to be screened for said inhibition to said plurality of containers, and (iii) adding a detectable, preferably a fluorescently labelled ligand to said containers, and (iv) incubating said DAX-1 protein, or said fragment, or derivative, or variant thereof, and said compound or plurality of compounds, and said detectable, preferably fluorescently labelled ligand, and (v) measuring the amounts of preferably fluorescence associated with said DAX-1 protein, or with said fragment, or derivative, or variant thereof, and (vi) determining the degree of inhibition by one or more of said compounds of binding of said ligand to said DAX-1 protein, or said fragment, or derivative, or variant thereof. It might be preferred to reconstitute said DAX-1 translation product, or fragment, or derivative, or variant thereof into artificial liposomes to generate the corresponding proteoliposomes to determine the inhibition of binding between a ligand and said DAX-1 translation product. Methods of reconstitution of DAX-1 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of a gene coding for DAX-1, or a fragment, or derivative, or variant thereof. One example of a fluorescent binding assay, in this case based on the use of carrier particles, is disclosed and described in patent application WO 00/52451. A further example is the competitive assay method as described in patent WO 02/01226. Preferred signal detection methods for screening assays of the instant invention are described in the following patent applications: WO 96/13744, WO 98/16814, WO 98/23942, WO 99/17086, WO 99/34195, WO 00/66985, WO 01/59436 and WO 01/59416.

In one further embodiment, the present invention provides a method for producing a medicament comprising the steps of (i) identifying a compound as an inhibitor of binding between a ligand and a gene product of a gene coding for DAX-1 by the aforementioned inhibitory binding assay and (ii) admixing the compound with a pharmaceutical carrier. However, said compound may also be identifiable by other types of screening assays.

In another aspect, the invention features an assay for testing a compound, preferably for screening a plurality of compounds to determine the degree of binding of said compounds to DAX-1 protein, or to a fragment, or derivative, or variant thereof. Said screening assay comprises (i) adding a liquid suspension of said DAX-1 protein, or a fragment, or derivative, or variant thereof, to a plurality of containers, and (ii) adding a detectable, preferably a fluorescently labelled compound or a plurality of detectable, preferably fluorescently labelled compounds to be screened for said binding to said plurality of containers, and (iii) incubating said DAX-1 protein, and said detectable, preferably fluorescently labelled compound or detectable, preferably fluorescently labelled compounds, and (iv) measuring the amounts of preferably the fluorescence associated with said DAX-1 protein, and (v) determining the degree of binding by one or more of said compounds to said DAX-1 protein, In this type of assay it might be preferred to use a fluorescent label. However, any other type of detectable label might also be employed. Also in this type of assay it might be preferred to reconstitute a DAX-1 translation product or fragment, or derivative, or variant thereof into artificial liposomes as described in the present invention. Said assay methods may be useful for the identification of novel compounds as well as for evaluating compounds which have been improved or otherwise optimized in their ability to bind to DAX-1.

In one further embodiment, the present invention provides a method for producing a medicament comprising the steps of (i) identifying a compound as a binder to a gene product of a gene coding for DAX-1 by the aforementioned binding assays and (ii) admixing the compound with a pharmaceutical carrier. However, said compound may also be identifiable by other types of screening assays.

In another embodiment, the present invention provides for a medicament obtainable by any of the methods according to the herein claimed screening assays. In one further embodiment, the instant invention provides for a medicament obtained by any of the methods according to the herein claimed screening assays.

The present invention features a protein molecule and the use of said protein molecule as shown in SEQ ID NO. 1, said protein molecule being a translation product of the gene coding for DAX-1, as diagnostic target for detecting a neurodegenerative disease, preferably Alzheimer's disease.

Furthermore, the present invention features a protein molecule and the use of said protein molecule as shown in SEQ ID NO. 1, said protein molecule being a translation product of the gene coding for DAX-1, as screening target for reagents or compounds preventing, or treating, or ameliorating a neurodegenerative disease, preferably Alzheimer's disease.

The present invention features the use of an antibody which is specifically immunoreactive with an immunogen, wherein said immunogen is a translation product of a gene coding for DAX-1. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of a gene coding for DAX-1, or fragments, or derivatives, or variants thereof.

In a preferred embodiment of the present invention, said antibodies can be used for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell. Preferably, the pathological state relates to a neurodegenerative disease, in particular to Alzheimer's disease. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

Figures 1 and 2 illustrate the differential expression of the human DAX-1 gene in AD brain tissues by quantitative RT-PCR analysis. Quantification of RT-PCR products from RNA samples collected from the frontal cortex (F) and the temporal cortex (T) of AD patients (Figure 1a) and samples from the frontal cortex (F) and the hippocampus (H) of AD patients (Figure 2a) was performed by the LightCycler rapid thermal cycling technique. Likewise, samples of healthy, age-matched control individuals were compared (Figure 1b for frontal cortex and temporal cortex, Figure 2b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. Note that the amplification kinetics of DAX-1 cDNAs from both, the frontal and temporal cortices of a normal control individual, and from the frontal cortex and hippocampus of a normal control individual, respectively, during The exponential phase of the reaction are juxtaposed (Figures 1b and 2b, arrowheads), whereas in Alzheimer's disease (Figures 1a and 2a, arrowheads) there is a significant separation of the corresponding curves, indicating a differential expression of the gene coding for DAX-1 in the respective analyzed brain regions, indicating a dysregulation, preferably an upregulation of a transcription product of the human DAX-1 gene, or a fragment, or derivative, or variant thereof, in the temporal cortex relative to the frontal cortex, and in the hippocampus relative to the frontal cortex.

Figure 3 shows the analysis of absolute mRNA expression of DAX-1 by comparison of control and AD stages using statistical method of the median at 98%-confidence level. The data were calculated by defining control groups including subjects with either Braak stages 0 to 1, Braak stages 0 to 2, or Braak stages 0 to 3 which are compared with the data calculated for the defined AD patient groups including Braak stages 2 to 6, Braak stages 3 to 6 and Braak stages 4 to 6, respectively. Additionally, three groups including subjects with either Braak stages 0 to 1, Braak stages 2 to 3 and Braak stages 4 to 6, rspectively, were compared with each other. A significant difference was detected comparing frontal cortex (F) and inferior temporal cortex (T) of AD patients and of control persons with each other. Said difference reflects an upregulation of DAX-1 in the temporal cortex of AD patients relative to the temporal cortex of control persons which is prominent comparing the Braak stages 0-3 with Braak stages 4-6 with each other. In frontal cortices a comparable upregulation cannot be observed. The differences reflect as well a down-regulation of DAX-1 in the frontal cortex of AD patients compared to the frontal cortex of control group subjects. The Braak stages correlate with the progressive course of AD disease which, as shown in the instant invention, is associated with an increasing difference in the regulation, the level and the activity of DAX-1 as described above. Said significant differences were observed already at Braak stage 3.

Figure 4 discloses SEQ ID NO. 1, the full-length amino acid sequence of the human DAX-1 protein, comprising 470 amino acids, as defined by the SwissProt accession number Q96F69.

Figure 5 shows SEQ ID NO. 2, the nucleotide sequence of the human DAX-1 cDNA, comprising 2022 nucleotides, as defined by the Genbank accession number S74720.

Figure 6 shows the nucleotide sequence of SEQ ID NO. 3, the coding sequence (cds) of the human DAX-1 gene, comprising 1413 nucleotides (nucleotides 235-1647 of SEQ ID NO. 2).

Figure 7 depicts the sequence alignment of the primers used for DAX-1 transcription level profiling by quantitative RT-PCR with the corresponding clippings of the nucleotide sequence of SEQ ID NO. 2 (nucleotides 1356 to 1407).

Figure 8 lists the expression levels of DAX-1 in the temporal cortex relative to the frontal cortex in fifteen AD patients, herein identified by internal reference numbers P010, P011, P012, P014, P016, P017, P019, P038, P040, P041, P042, P046, P047, P048, P049 (0.58-7.53 fold, values according to the formula described below) and twentyfour age-matched control individuals, herein identified by internal reference numbers C005, C008, C011, C012, C014, C025, C026, C027, C028, C029, C030, C031, C032, C033, C034, C035, C036, C038, C039, C041, C042, DE02, DE05, DE07 (0.36-2.26 fold, values according to the formula described below). For an up-regulation in the temporal cortex, the values shown are calculated according to the formula described herein (see below) and in case of an up-regulation in the frontal cortex the reciprocal values are calculated, respectively. The bar diagram visualizes individual natural logarithmic values of the temporal to frontal cortex, In(IT/IF), and of the frontal to temporal cortex regulation factors, In(IF/IT), in different Braak stages (0 to 6).

Figure 9 lists the gene expression levels in the hippocampus relative to the frontal cortex for the DAX-1 gene in six Alzheimer's disease patients, herein identified by internal reference numbers P010, P011, P012, P014, P016, P019 (1.14 to 13.61 fold) and three healthy, age-matched control individuals, herein identified by internal reference numbers C004, C005, C008 (0.08 to 2.23 fold). The values shown are calculated according to the formula described herein (see below). The scatter diagram visualizes individual logarithmic values of the hippocampus to frontal cortex regulation ratios (log (ratio HC/IF)) in control samples (dots) and in AD patient samples (triangles).

Figure 10 depicts a Western blot image of total human brain extracts labeled with a rabbit polyclonal anti-DAX-1 antibody (K-17, Santa Cruz). M: molecular weight marker (Santa Cruz Marker); Lanes 1 and 2: human brain (cerebral cortex) total protein extract samples derived from two different tissue donors. The arrow indicates a major band at about 52 kDa, which corresponds to the predicted molecular weight of the full length DAX-1 protein.

Figure 11 exemplarily depicts micrographs digitally taken from sections of the inferior temporal gyrus from a control donor (Control T, C030, C027, Figures 11A, C) and from Alzheimer patients (Patient T, P011, P016, Figures 11B, D) immunolabeled with rabbit polyclonal anti-DAX-1 antiserum (green signals) (magnification 40x). Immunoreactivity of DAX-1 was observed in all neurons, where it is detected predominantly in the nuclei. Neurons (neuronal nuclei and somata) are stained with an antibody against the neuron-specific marker NeuN (red signal). The nucleus is stained with DAPI (blue signal). Weak DAX-1 immunoreactivity is found in astrocytes, where it is largely absent from microglia, oligodendrocytes and myelin. The data exemplarily shown here clearly indicate that the level of intensity and quantity of neuronal immunoreactivity of the DAX-1 protein is reduced in the inferior temporal cortex from patients (Figures 11B and 11D) as compared to the inferior temporal cortex from control persons (Figures 11A and 11C). DAX-1 expression is decreased in neurons in AD brain in all samples analyzed. The neuronal DAX-1 immunoreactivity which is detected in neurons of control samples (Figures 11A, C, arrow) is no longer detectable in any of the AD-patient samples examined (Figure 11B, D). Additionally, in comparison with sections from controls, astrocytic DAX-1 immunoreactivity (more clearly observed in the white matter, Figures 11B, D) is increased in temporal specimens from patients. The quantitative and qualitative astrocytic expression of DAX-1 protein reflects the progression of Alzheimer's disease (increased and strong homogeneous nuclear and cytoplasmic staining of DAX-1 with increasing Braak stages of AD observed). Cerebral cortex (CT); White matter (WM)

Figure 12 shows the immunofluorescence analysis of $H4APP_{SW}$ control cells and H4APPsw cells stably over-expressing the myc-tagged DAX-1 protein (H4APPsw-DAX-1-myc). The DAX-1-myc protein was detected with a rabbit anti-myc antibody (Mobitec) and a Cy3-conjugated anti-rabbit antibody (Amersham) (Figures 12A and B). The cellular nucleus was stained with DAPI (Figures 12C and D). The overlay analysis indicate that the DAX-1-myc protein is localized to the nucleus (Figure 12E) and is over-expressed in more than 50% of the H4APPsw-DAX-1-myc transduced cells as compared to the H4APPsw control cells (Figure 12F).

Figure 13 indicates that the over-expression of DAX-1-myc protein renders H4APPsw cells to be more resistant of trophic factor deprivation than H4APPsw control cells. Both cell types have been incubated for 40 hours in cell culture media that contained serum concentrations ranging from 0 to 7.5%. The cellular response to trophic factor deprivation was determined by applying the REDOX indicator AlamarBlue (BioSource) and measuring the relative fluorescence (RFU). The % toxicity was calculated according to the formula as given below (Example (vii)). The graph represents a toxicity calculation obtained from one representative experiment.

Figure 14 shows the detection of DAX-1 expression in two different DAX-1 transgenic fly lines under the control of gmr-GAL4 by Western blotting. Genotypes used were: *w1118; w; UAS-DAX-1#1/+; gmr-GAL4/+; w; UAS-DAX-1#18/gmr-GAL4.* The blot was probed with antibody sc841 (Santa Cruz; anti-DAX1 antibody; 1:200; rabbit polyclonal) and E7 (anti-beta-tubuline; Developmental Studies Hybridoma Bank; 1 :1000; mouse monoclonal). The DAX-1 protein is detectable as a faint bank located below a strong cross-reaction band which is also visible in non-transgenic control flies (red asterisk). The beta-tubuline signal serves as loading control.

Figure 15 depicts the expression of DAX-1 under the control of gmr-GAL4. 10μm Cryostat adult brain sections were stained with a photoreceptor cell specific antibody 24B10 at 1 day and 8 days after eclosion. The expression of DAX-1 in the adult retina is not interfering with the integrity of photoreceptor cells, no pathological phenotype is visible. Genotypes used were: *w1118; w; UAS-DAX-1#1/+; gmr-GAL4/+ - w; UAS-DAX-1#18/gmr-GAL4 - w; UAS-DAX-1#28/+; gmr-GAL4/-+.* Re: retina; La: lamina; Me: medulla.

Figure 16 shows 'Toluidine Blue' staining of plastic sections of age-matched 16 days old flies expressing hAPP/hBACE or hAPP/hBACE in combination with DAX-1#1 (Figure 16 A). Rhabdomeres of photoreceptor cells appear as blue dots within each ommatidium (red arrow head). Figure 16B and C depicts the 10μm cryostat sections of the adult retina of hAPP/hBACE expressing control flies and hAPP/hBACE in combination with DAX-1#1 (Figure 16B, 10-12 days post eclosion) or DAX-1#28 (Figure 16C, 4 days post eclosion). Sections were stained with the photoreceptor cell specific antibody 24B10. As shown Photoreceptor cell degeneration is significantly delayed by co-expression of DAX-1 as judged by the diameter of the retina (indicated by white arrows).

Figure 17 pictures thioflavin S positive plaques (indicated by arrows) on paraffin sections through the retina of flies expressing hAPP/hBACE/DPsnL235P (Figure 17A) or hAPP/hBACE/DPsnL235Pm in combination with DAX-1#1 (Figure 17B). The onset of plaque deposition is observed in 35 days old control flies whereas the onset of plaque deposition is delayed by 10 days in flies co-expressing DAX-1#1. Genotypes used were:. *w; UAS-hBACE, UAS-*

*hAPP/+; gmr-GAL4, UAS-DPsnL235P/+ and w; UAS-hBACE, UAS-hAPP/UAS-DAX-1#1; gmr-GAL4, UAS-DpsnI235P/+.* The stars indicate locations where amyloid plaques were present before sectioning, as the plaques are often displaced by the sectioning procedure.

Figure 18 depicts 10μm cryostat sections of the adult retina of bTAU expressing control flies and flies expressing bTAU in combination with DAX-1#1 stained at 2 days and at 11 days post eclosion, respectively. The sections were stained with a photoreceptor cell specific antibody 24B10. The co-expression of DAX-1 rescues the photoreceptor cell degeneration which is induced by TAU, as judged by the diameter of the retina (white arrows) (shown in 11 days old flies). Genotypes used were: *w; gmr-GAL4/UAS-bTAU* and *w; UAS-DAX1#1/+; gmr-GAL4/UAS-bTAU.* Re: retina; La: lamina; Me: medulla.

Figure 19 schematically depicts the targeting strategy used to generate transgenic mice expressing human DAX-1. The targeting vector is composed of the transgene and Rosa26 homology sequences in order to integrate the transgene into the Rosa26 gene locus (mouse Rosa beta geo 26 gene). The short homology arm sequence (SA) consists of approximately 1.1 kb, the long homology arm sequence (LA) consists of about 4.3 kb. The three exons of the Rosa26 gene are indicated with roman numbers (I-III). The transgene is composed of the open reading frame of the human DAX-1 (ORF DAX-1) (SEQ ID NO. 3 and/or a fragment of SEQ ID NO. 2) which is located in the murine Thy1.2 expression cassette containing regulatory sequences of the brain-specific Thy1.2 gene. 5'prime of the transgene a Neomycin resistance gene (Neo, Neomycinphosphotransferase) and a splice acceptor are located allowing expression of the Neo selection marker driven by the Rosa promoter from the endogenous Rosa26 gene. 3'prime of the Neo marker a poly A signal sequence (pA) will guarantee termination of the transcription.

Figure 20 represents a Southern Blot analysis of targeted ES cell clones. Genomic DNA from six different ES cell clones was digested with BglI and subjected to Southern Blot analysis. The positions of the BglI restriction sites as well as the binding site of the probe (sequence hybridyzing with exon II of the Rosa26 gene) are indicated in the outline of the mouse wildtype allele (WT) and the DAX-1 targeted homologous recombinated allele (HR) (Figure 20A). The three exons of the Rosa26 gene are indicated with roman numbers (I-III). The Southern Blot in Figure 20B shows five targeted ES cell clones (indicated by the numbers A10, B2, B10, C6, C9) and the wildtype allele as control (WT). All five targetd ES cell clones harbor the homologous recombinated allele (HR) which can be identified by the presence of a band at 10.1 kb (HR) in addition to the respective band at 6.7 kb which represents the wildtype allele (WT).

EXAMPLES:

(i) Brain tissue dissection from patients with AD:

Brain tissues from AD patients and age-matched control subjects were collected within 5 hours post-mortem and immediately frozen on dry ice. Sample sections from each tissue were fixed in paraformaldehyde for histopathological confirmation of the diagnosis. Brain areas for differential expression analysis were identified and stored at -80 °C until RNA extractions were performed.

(ii) Isolation of total mRNA:

Total RNA was extracted from post-mortem brain tissue by using the RNeasy kit (Qiagen) according to the manufacturer's protocol. The accurate RNA concentration and the RNA quality were determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler technology as described in the supplied protocol by the manufacturer (Roche).

(iii) Quantitative RT-PCR analysis:

The differential expression levels of the DAX-1 gene in the temporal cortex versus frontal cortex and in the hippocampus versus frontal cortex were analyzed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than endpoint readout. The ratios of DAX-1 cDNAs from the temporal cortices of AD patients and of healthy age-matched control individuals, from the frontal cortices of AD patients and of healthy age-matched control individuals, from the hippocampi of AD patients and of healthy age-matched control individuals, and the ratios of DAX-1 cDNAs from the temporal cortex and frontal cortex of AD patients and of healthy age-matched control individuals, and the ratios of DAX-1 cDNAs from the hippocampus and frontal cortex of AD patients and of healthy age-matched control individuals, respectively, were determined (relative quantification).
The mRNA expression profiling between frontal cortex tissue (F) and inferior temporal cortex tissue (T) of DAX-1 has been analyzed in four up to nine tissues per Braak stage. Because of the lack of high quality tissues from one donor with Braak 3 pathology, tissues of one additional donor with Braak 2 pathology were included, and because of the lack of high quality tissues from one donor with Braak 6 pathology, tissue samples of one additional donor with Braak 5 pathology were included.
For the analysis of the profiling, two general approaches have been applied. Both comparative profiling studies, frontal cortex against inferior temporal cortex as well as control against AD patients, which contribute to the complex view of the relevance of DAX-1 in AD physiology, are shown in detail below. 1) Relative comparison of the mRNA expression between frontal cortex tissue and inferior temporal cortex tissue of controls and of AD patients.
This approach allowed to verify that DAX-1 is either involved in the protection of the less vulnerable tissue (frontal cortex) against degeneration, or is involved in or enhances the process of degeneration in the more vulnerable tissue (inferior temporal cortex).
First, standard curves were generated to determine the efficiency of the PCR with primers for the DAX-1 gene:

5'-TACCAAGGAGTACGCCTACCTCA-3' (SEQ ID NO. 2, nucleotides 1356-1378; SEQ ID NO. 3, nucleotides 1122-1144) and
5'-CACGTCCGGGTTAAAGAGCA-3' (SEQ ID NO. 2, nucleotides1407-1388; SEQ ID NO. 3, nucleotides 1173-1153);

PCR amplification (95 °C and 1 sec, 56 °C and 5 sec, and 72 °C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and, depending on the primers used, additional 3 mM $MgCl_2$. Melting curve analysis revealed a single peak with no visible primer dimers at approximately 83.5°C. Quality and size of the PCR product were determined with the DNA LabChip system (Agilent 2100 Bioanalyzer, Agilent Technologies). A single peak at the expected size of 52 bp for the DAX-1 was observed in the electropherogram of the sample.
In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTTTGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'-TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'-TGGAACGGTGAAGGTGACA-3' and 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'-GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for DAX-1 and the five reference standard genes. The slopes and the intercepts of the standard curves (i.e. linear regressions) were calculated for all genes. In a second step, cDNAs from frontal cortices of AD patients and of healthy control individuals, from temporal cortices of AD patients and of healthy control individuals, from hippocampi of AD patients and of healthy control individuals, and cDNAs from the frontal cortex and the temporal cortex of AD patients and of control individuals and from the frontal cortex and the hippocampus of AD patients and of control individuals, respectively, were analyzed in parallel and normalized to cyclophilin B. The $C_t$ values were measured and converted to ng total brain cDNA using the corresponding standard curves:

$$10 \wedge ( (C_t \text{ value - intercept}) / \text{slope} ) \quad [\text{ng total brain cDNA}]$$

The values for temporal and frontal cortex DAX-1 cDNAs, the values for hippocampus and frontal cortex DAX-1 cDNAs, and the values from the frontal cortex DAX-1 cDNAs of AD patients (P) and control individuals (C), and the values for temporal cortex DAX-1 cDNAs of AD patients (P) and of control individuals (C), were normalized to cyclophilin B, and the ratios were calculated according to formulas:

$$\text{Ratio} = \frac{\text{DAX-1 temporal [ng] / cyclophilin B temporal [ng]}}{\text{DAX-1 frontal [ng] / cyclophilin B frontal [ng]}}$$

$$\text{Ratio} = \frac{\text{DAX-1 hippocampus [ng] / cyclophilin B hippocampus [ng]}}{\text{DAX-1 frontal [ng] / cyclophilin B frontal [ng]}}$$

$$\text{Ratio} = \frac{\text{DAX-1 (P) temporal [ng] / cyclophilin B (P) temporal [ng]}}{\text{DAX-1 (C) temporal [ng] / cyclophilin B (C) temporal [ng]}}$$

$$\text{Ratio} = \frac{\text{DAX-1 (P) frontal [ng] / cyclophilin B (P) frontal [ng]}}{\text{DAX-1 (C) frontal [ng] / cyclophilin B (C) frontal [ng]}}$$

In a third step, the set of reference standard genes was analyzed in parallel to determine the mean average value of the AD patient to control person temporal cortex ratios, of the AD patient to control person frontal cortex ratios, and of the temporal to frontal ratios of AD patients and control persons and the hippocampi to frontal ratios of AD patients and control persons, respectively, of expression levels of the reference standard genes for each individual brain sample. As cyclophilin B was analyzed in step 2 and step 3, and the ratio from one gene to another gene remained constant in different runs, it was possible to normalize the values for DAX-1 to the mean average value of the set of reference standard genes instead of normalizing to one single gene alone. The calculation was performed by dividing the respective ratio shown above by the deviation of cyclophilin B from the mean value of all housekeeping genes. The results of such quantitative RT-PCR analysis and the respective calculated values for the gene coding for the DAX-1 are shown in Figures 1 and 8 and in Figures 2 and 9.

2) Comparison of the mRNA expression between controls and AD patients.

For this analysis it was proven that absolute values of real-time quantitative PCR (Lightcycler method) between different experiments at different time points are consistent enough to be used for quantitive comparisons without usage of calibrators. Cyclophilin was used as a standard for normalization in any of the qPCR experiments for more than 100 tissues. Between others it was found to be the most consistently expressed housekeeping gene in our normalization experiments. Therefore a proof of concept was done by using values that were generated for cyclophilin.

First analysis used cyclophilin values from qPCR experiments of frontal cortex and inferior temporal cortex tissues from three different donors. From each tissue the same cDNA preparation was used in all analyzed experiments. Within this analysis no normal distribution of values was achieved due to small number of data. Therefore the method of median and its 98 %-conficence level was applied. This analysis revealed a middle deviation of 8.7 % from the median for comparison of absolute values and a middle deviation of 6.6 % from the median for relative comparison.

Second analysis used cyclophilin values from qPCR experiments of frontal cortex and inferior temporal cortex tissues from two different donors each, but different cDNA preparations from different time points were used. This analysis revealed a middle deviation of 29.2 % from the median for comparison of absolute values and a middle deviation of 17.6 % from the median for relative comparison. From this analysis it was concluded, that absolute values from qPCR experiments can be used, but the middle deviation from median should be taken into further considerations. A detailed analysis of absolute values for DAX-1 was performed. Therefore, absolute levels of DAX-1 were used after relative normalization with cyclophilin. The median as well as the 98 %-confidence level was calculated for the control group (Braak 0 - Braak 3) and the patient group (Braak 4 - Braak 6), respectively. The same analysis was done redefining the control group (Braak 0 - Braak 2) and the patient group (Braak 3 - Braak 6) as well as redefining the control group (Braak 0 - Braak 1) and the patient group (Braak 2 - Braak 6). The latter analysis was aimed to identify early onset of mRNA expression differences between controls and AD patients. In another view of this analysis, three groups comprising Braak stages 0-1, Braak stages 2-3, and Braak stages 4-6, respectively, were compared to each other in order to identify tendencies of gene expression regulation as well as early onset differences. Said analysis as described above is shown in Figure3.

(iv) Immunohistochemistry:

For immunofluorescence staining of DAX-1 in human brain, and for the comparison of AD-affected tissue with control tissues, post-mortem fresh-frozen frontal and temporal forebrain specimens from donors comprising patients with clinically diagnosed and neuropathologically confirmed Alzheimer's disease at various Braak stages (P038, P016, P011, P017; Braak 4, 5, 6) as well as age-matched control individuals without Alzheimer (C027, C030; Braak 0, 1), were cut at 14 μm thickness using a cryostat (Leica CM3050S). The tissue sections were air-dried at room temprature and fixed in acetone for 10 min. After washing in PBS, the sections were pre-incubated with blocking buffer (10% normal horse serum, 0.2% Triton X-100 in PBS) for 30 min and then incubated with affinity-purified anti-DAX-1 rabbit polyclonal antibodies (1:20 diluted in blocking buffer; Santa Cruz Biotechnology, sc-13064 (H-330) or sc-841 (K-17), Heidelberg, Germany) overnight at 4°C. After rinsing three times in 0.1% Triton X-100/PBS, the sections were incubated with FITC-conjugated goat anti-rabbit IgG antiserum (Jackson/Dianova, No.111-096-045; 1 :150 diluted in 1% BSA/PBS) for 2 hours at room temperature and then again washed in PBS. Staining of the neuronal cells was performed as described above using a mouse monoclonal antibody against the neuronal specific marker NeuN (Chemicon, MAB377, dilution 1:200) and a secondary Cy3-conjugated goat anti-mouse antibody (Dianova, 115-166-062, dilution 1:600). Staining of the nuclei was performed by incubation of the sections with 5μM DAPI in PBS for 3 min. In order to block the autofluoresence of lipofuscin in human brain, the sections were treated with 1% Sudan Black B in 70% ethanol for 2-10 min at room temperature and then sequentially dipped in 70% ethanol, destilled water and PBS. The sections were coverslipped with 'Vectashield' mounting medium (Vector Laboratories, Burlingame, CA). Microscopic images were obtained using dark field epifluorescence and bright field phase contrast illumination conditions (IX81, Olympus Optical). Microscopic images were digitally captured with a PCO SensiCam and analyzed using the appropriate software (AnalySiS, Olympus Optical) (see Figure 11).

(v) Immunoblotting:

Brain tissue (0.2 g) was homogenized in 1 ml RIPA buffer (150 mM sodium chloride, 50 mM tris-HCl, pH7.4, 1 mM ethylenediamine-tetraacetic acid, 1 mM phenylmethylsulfonyl flouride, 1% Triton X-100, 1% sodium deoxycholic acid, 1% sodium dodecylsulfate, 5 μg/ml of aprotinin, 5 μg/ml of leupeptin) on ice. After centrifuging twice for 5 min at 3000 rpm at 4°C, the supernatant was diluted fivefold in SDS-loading buffer. Aliquots of 12 μl of the diluted sample were resolved by SDS-PAGE (8% polyacrylamide) and transferred to PVDF Western Blotting membranes (Boehringer Mannheim). The blots were probed with anti-DAX-1 rabbit polyclonal antibodies (Santa Cruz Biotechnology, sc-13064 (H-330) or sc-841 (K-17), Heidelberg) followed by horseradish peroxidase-coupled goat anti-rabbit IgG antiserum (Santa Cruz sc-2030, diluted 1:5000) and developed with the ECL chemoluminescence detection kit (Amersham Pharmacia) (Figure 10).

(vi) Immunofluorescence Analysis (IF):

For the immunofluorescence staining of DAX-1 protein in cells, a human neuroglioma cell line was used (H4 cells) which stably expresses the human-APP695 isoform carrying the Swedish mutation (K670N, M671 L) (H4APPsw cells). The H4APPsw cells were transduced with a pFB-Neo vector (Stratagene, #217561, 6.6 kb) containing the coding sequence of DAX-1 (DAX-1cds) (SEQ ID NO. 3, 1413 bp) (pFB-Neo-DAX-1cds-myc, DAX-1 vector, 8034 bp) and a myc-tag. For the generation of the DAX-1 vector, the DAX-1cds-myc sequence was introduced into the EcoRI/XhoI restriction sites of the multiple cloning site (MCS) of the pFB-Neo vector. For transduction of the H4APPsw cells with the DAX-1 vector the retroviral expression system ViraPort from Stratagene was used.

The myc-tagged DAX-1 over-expressing cells (H4APPsw-DAX-1-myc) were seeded onto glass cover slips in a 24 well plate (Nunc, Roskilde, Denmark; #143982) at a density of $5x10^4$ cells and incubated at 37°C at 5% $CO_2$ over night. To fix the cells onto the cover slip, medium was removed and chilled methanol (-20°C) was added. After an incubation period of 15 minutes at -20°C, methanol was removed and the fixed cells were blocked for 1 hour in blocking solution (200μl PBS/ 5% BSA/ 3% goat serum) at room temperature. The first antibody (polyclonal anti-myc antibody, rabbit, 1:500, Mobitec) and DAPI (DNA-stain, 0.05μg/ml, 1:1000) in PBS / 1% goat serum was added and incubated for 1 hour at room temperature. After removing the first antibody, the fixed cells were washed 3 times with PBS for 5 minutes. The second antibody (Cy3-conjugated anti-rabbit antibody, 1:1000, Amersham Pharmacia, Germany) was applied in blocking solution and incubated for 1 hour at room temperature. The cells were washed 3 times in PBS for 5 minutes. Coverslips were mounted onto microscope slides using Permafluor (Beckman Coulter) and stored over night at 4°C to harden the mounting media. Cells were visualized using microscopic dark field epifluorescence and bright field phase contrast illumination conditions (IX81, Olympus Optical). Microscopic images (Figure 12) were digitally captured with a PCO SensiCam and analysed using the appropriate software (AnalySiS, Olympus Optical).

(vii) Cytotoxicity/ Viability Assay:

To analyse the effect of trophic factor deprivation on DAX-1 protein over-expressing cells (DAX-1 cells described above in Example (vi)), H4APPsw control and H4APPsw-DAX-1-myc over-expressing cells were seeded with a density of $1.5x10^4$ cells/ml in 96 well plates in DMEM (Simga), 10% FCS (Gibco) and Penicillin/Streptomycin (Gibco). After incubating over night at 37°C under $CO_2$ conditions (8.5%), medium was exchanged against DMEM with Penicillin/ Streptomycin. As a positive control for toxicity, 12 concentrations of chloroquine (Sigma) ranging from 0.06-10 mg/ml were added to control cells and to H4APPsw-DAX-1-myc over-expressing cells (in triplicate). As a negative control for toxicity, DMEM (Simga), 10% FCS (Gibco) and Penicillin/Streptomycin (Gibco) was applied to 24 wells of control cells and H4APPsw-DAX-1-myc over-expressing cells. For trophic factor deprivation, increasing serum concentrations (0, 0.3, 0.6, 1.25, 2.5, 5% and 7.5%) in DMEM, Penicillin/Steptomycin were added to control cells and H4APPsw-DAX-1-myc over-expressing cells (in triplicate). After incubation for 40 hours at 37°C under $CO_2$ conditions (8.5%), the REDOX indicator AlamarBlue (BioSource) was added and the relative fluorescence (RFU) was measured 4 hours later at 544nm (excitation) and 590nm (emission) using a BMG Fluostar reader. The % of toxicity was calculated with RFU mean values using the following formula:

$$\%\ \text{Toxicity} = \frac{100 \times (\text{RFU negative control} - \text{RFU toxic stimuli})}{(\text{RFU negative control} - \text{RFU positive control})}$$

Graphs have been determined by using GraphPad Prism (non-linear regression curve fit with sigmoidal dose response and variable slope).

(viii) Generation of transgenic *Drosophila melanogaster:*

Human BACE transgenic flies and human DAX-1 transgenic flies were generated according to Greeve et al. (Greeve et al., J. Neurosci. 2004, 24: 3899-3906) and as described in the present invention. A 1576 bp EcoRI/NotI fragment of the DAX-1 cDNA (SEQ ID NO. 2) containing the entire open reading frame of DAX-1 (SEQ ID NO.3) was cloned into the EcoRI/NotI restriction sites of the vector pUAST to ensure an oriented subcloning of the DAX-1 insert downstream of the UAS-binding sites (Brand and Perrimon, Development 1993, 118: 401-15). P-element-mediated germline transformation was performed as described by Spradling and Rubin (Rubin and Spradling, Science 1982, 218: 348-53; Spradling and Rubin, Science 1982, 218: 341-7). Twentysix independent human DAX-1 transgenic fly lines were generated and three different lines were used for the analysis.
Human APP and *Drosophila* Presenilin transgenic flies, the UAS-APP695II and the UAS-DPsn-mutants (L235P), were kindly provided by R. Paro and E. Fortini (Fossgreen et al., Proc Natl Acad Sci U S A 1998, 95: 13703-8; Ye and Fortini, J Cell Biol 1999, 146: 1351-64). The UAS-bovine TAU transgenic fly line was provided by S. Schneuwly (Ito et al., Cell Tissue Res. 1997, 290: 1-10). The actin-GAL4 line was obtained from the Bloomington stock center. The gmr-GAL4 line from F. Pignoni was used to achieve the eye-specific expression of the transgenes. Genetic crosses were set up on standard *Drosophila* culture medium at 25°C. Genotypes used were: w; UAS-hAPP$_{695}$, UAS-hBACE437/CyO; gmr-GAL4/Tm3 - w; UAS-hAPP$_{695}$, UAS-hBACE437/CyO; gmr-GAL4,UAS-DPsnL235P/Tm3 - w; UAS-DAX-1#1 (2$^{nd}$ chromosome, viable insertion) - w; UAS-DAX-1#18 (3$^{rd}$ chromosome, viable insertion) - w; UAS-DAX-1#28/CyO (2$^{nd}$ chromosome, lethal insertion For immunohistochemical and histological analysis the adult flies were immunostained and prepared according to the following methods. For immunostaining adult flies were fixed in 4% paraformaldehyde for 3 hours, washed in 1 x PBS and transferred to 25% sucrose for an overnight incubation at 4°C. Flies were decapitated with a razor blade, and the heads were imbedded in Tissue Tek (Sakura) and snap frozen. 10μm horizontal frozen sections were prepared on a cryostat (Leica CM3050S). Immunostaining was done with the Vectastain Elite kit (Vector Laboratories) according to the instructions of the manufacturer. The following primary antibody was used: mouse monoclonal antibody 24B10 (alpha-chaoptin, 1:5) provided by the Developmental Studies Hybridoma Bank. Paraffin sections of adult heads were prepared as described by Tschäpe J.-A. et al. (EMBO J. 2002, 21: 6367-6376). For thioflavin S staining sections were counterstained for 5 minutes in Mayers Hemalum (Sigma), rinsed for 10 minutes in tap water and stained for 3 minutes in 1% thioflavin S (Sigma) watery solution. Slides were rinsed in several changes of distilled water, incubated for 15 minutes in 1% acetic acid, rinsed in tap water and mounted in Vectashield mounting medium (Vector laboratories). Slides were analyzed under an Olympus BX51 fluorescence microscope (430 nm excitation, 550 nm emission).

For the protein analyses by western blotting, fly heads were homogenized in 1xPBS, 5mM EDTA, 0.5% Triton X-100 and a protease-inhibitor mix Complete (Roche Applied Science). Equal amounts of protein were separated by 10% SDS-PAGE, transferred to Immobilon membranes (Millipore GmbH), blocked in 5% low fat milk for two hours at room temperature and incubated with the monoclonal antibody 22C11 (APP N-terminal specific, Chemicon international), rabbit polyclonal sc841 (Santa Cruz) and monoclonal antibody alpha-beta-tubuline (E7, Developmental Studies Hybridoma Bank). Bound antibodies were detected with goat anti-mouse peroxidase conjugated secondary antibodies (Dianova).

For immunoprecipitation fly heads were homogenized as described above and lysates were treated as described in the antibodies protocol guide from Clontech. The antibody mab 6E10 (alpha-Abeta1-16, Signet Pathology Systems) was used for immunoprecipitation. Samples were separated on 10-20% gradient Novex Tris-Tricine gels (Invitrogen) and blotted onto Protran BA 79 Cellulose nitrate membranes (0.1μm, Schleicher/Schuell, Dassel, Germany). Detection of beta-amyloid was performed as described (Ida et al., J Biol Chem 1996, 271: 22908-14) using mab 6E10 and goat anti-mouse peroxidase conjugated secondary antibody (Dianova).

To characterize the potential impact of human DAX-1 expression on the neuropathology associated with amyloidogenic processing of human APP (beta-amyloid precursor protein, hAPP) in transgenic flies, DAX-1 was co-expressed with hAPP$_{695}$ and human BACE (Beta site APP cleaving enzyme, hBACE) in the adult retina by using the eye-specific GAL4 line gmr-GAL4.

Transgenic expression of DAX-1 under the control of gmr-GAL4 was confirmed by Western blotting (see Figure 14). The DAX-1 specific antibody sc841 (Santa Cruz) detects a protein with an apparent molecular weight of 50kD. Two different transgenic fly lines were used (DAX-1#1 and DAX-1#18) for the analysis. Over-expression of DAX-1 in the adult retina has no impact on proper assembly and differentiation of photoreceptor cells of the adult retina as demonstrated in Figure 15. 10μm cryostat sections through the brain and retina of flies expressing DAX-1 under the control of gmr-GAL4 were stained with a photoreceptor cell specific antibody 24B10 and compared to non-transgenic control flies. Three different DAX-1 transgenic fly lines were used for the analysis and staining of photoreceptor cells was compared of age matched flies 1 and 8 days after eclosion.

Expression of hAPP and hBACE in the adult retina of *Drosophila* leads to age-dependent degeneration of photoreceptor cells (Greeve et al., J. Neurosci. 2004, 24: 3899-3906). Co-expression of DAX-1 leads to a prominent rescue of the degenerative phenotype, of photoreceptor cell degeneration in young (Figure 16C; 4 days old) and aging flies (Figure 16A and B; 16 and 12 days old flies) as demonstrated on 1μm plastic sections that were stained with Toluidine Blue (Figure 16A) and on cryostat sections that were stained with a photoreceptor cell specific antibody (Figure 16B and C). Two independent DAX-1 transgenic fly lines were used for this analysis (DAX1#1 and DAX1#28). Figure 16A demonstrates that co-expression of hAPP and hBACE leads to profound degeneration of photoreceptor cells in the retina of 16 days old flies (black arrow in Figure 16A points to ommatidium with missing photoreceptor cells) whereas in age-matched flies co-expressing DAX-1 the degeneration of photoreceptor cells is significantly delayed (red arrow in Figure 16A points to ommatidium with the full complement of photoreceptor cells).

Amyloid plaque deposition in the retina of hAPP/hBACE expressing flies is accelerated by the co-expression of mutant forms of Presenilin (Greeve et al., J. Neurosci. 2004, 24: 3899-3906). Therefore, we investigated the onset of Thioflavin S positive amyloid plaques in flies expressing hAPP/hBACE/DPsnL235P and compared them to flies expressing hAPP/hBACE/DPsnL235P and DAX-1#1. Figure 17 demonstrates a delayed onset of plaque-deposition in flies co-expressing DAX-1#1 (45 days after eclosion) in comparison to control flies which show plaque deposition already at 35 days post eclosion.
To further characterize the neuroprotective effect of DAX-1 on photoreceptor cell degeneration we investigated a second degenerative phenotype induced by expression of bovine TAU (bTAU). Eye-specific expression of bTAU under the control of gmr-GAL4again leads to severe age-dependent degeneration of photoreceptor cells in the adult retina (Figure 18, 2 days and 11 days old control flies expressing bTAU). Co-expression of DAX-1#1 again rescues photoreceptor cell degeneration (Figure 18, age-matched 2 days and 11 days old control flies co-expressing DAX-1#1). The rescue is even more evident in older flies.

(ix) Generation of transgenic mice expressing DAX-1 :

DAX-1 transgenic mice were generated to specifically expressing human DAX-1 cDNA in neuronal cells. For this purpose the open reading frame of the human DAX-1 (ORF DAX-1) cDNA, SEQ ID NO. 3 and/or a fragment of SEQ ID NO. 2, was cloned into the murine Thy1.2 expression cassette containing a brain-specific Thy-1 regulatory sequence (Thy-1.2 promotor and regulatory elements) to direct neuronal specific expression and thus, causes expression only in brain cells (Luthi and van der Putten, J. Neuroscience 1997, 17:4688-4699). The transgene was cloned into a Rosa26 targeting vector in order to integrate the sequences into the Rosa26 gene locus (mouse Rosa beta geo 26 gene on chromosome 6; Seibler et al., Nucleic Acids Research 2003, 31 :e12). 5' prime of the transgene a Neo selection marker was placed. The Neo cassette contains a splice acceptor allowing expression of the Neo selection marker driven by the Rosa promoter from the endogenous Rosa26 gene (Friedrich and Soriano, Genes Dev. 1991, 5:1513-1523; Zambrowicz et al., Proc Natl Acad Sci USA et al., 94:3789-3794). 3'prime of the Neo marker a poly A signal (pA) will stop transcription and the influence of the Rosa promoter. The targeting vector contains Rosa26 homology sequences from genomic 129S6 DNA to allow homologous recombination into the Rosa26 gene locus. The short homology arm (SA) consists of approximately 1.1 kb, the long homology arm (LA) consists of about 4.3 kb. The constructed targeting vector and the targeting strategy is shown in Figure 19.
The targeting vector as shown above was cloned and further, transfected into C57BL/6N mouse embryonal stem (ES) cells (mouse strain C57BL/6J). The transfection of ES cells was performed according to state of the art techniques (Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999). After homologous recombination targeted ES cell clones, which were identified by Southern Blot analysis (Figure 20) were injected into blastocysts to generate chimeric mice using standard techniques (Tymms and Kola, Gene Knockout Protocols, Humana Press 2001). After successful transmission of the germline of the chimeric mice generated, they were crossed to an Alzheimer's disease mouse model to produce transgenic DAX-1 mice on an Alzheimer's disease background.

## Claims

1. A method of diagnosing or prognosticating a neurodegenerative disease in a subject, or determining whether a subject is at increased risk of developing said disease, comprising determining a level and/or an activity of

   (i) a transcription product of a gene coding for DAX-1, and/or
   (ii) a translation product of a gene coding for DAX-1
   in a sample obtained from said subject and comparing said level and/or said activity to a reference value representing a known disease or health status, thereby diagnosing or prognosticating said neurodegenerative disease in said subject, or determining whether said subject is at increased risk of developing said neurodegenerative disease.

2. The method according to claim 1 wherein said neurodegenerative disease is Alzheimer's disease.

3. A genetically altered non-human animal comprising the non-native gene sequence of SEQ ID NO. 3 coding for DAX-1, wherein said non-human animal is a rodent, preferably a mouse, a rat or a guinea pig, or an invertebrate animal, preferably an insect, more preferably a fly such as the fly *Drosophila melanogaster,* wherein the expression of said gene results in said non-human animal having a reduced risk of developing symptoms similar to a neurodegenerative disease, in particular a reduced risk of developing symptoms similar to AD and/or which shows a reduction of AD symptoms and/or which has no AD symptoms due to an effect caused by the expression of the gene used to genetically alter said non-human animal.

4. Use of the genetically altered non-human animal according to claim 3 for screening, testing, and validating compounds, agents, and modulators in the development of diagnostics and therapeutics to treat neurodegenerative diseases, in particular Alzheimer's disease.

5. An in vitro assay for screening for a modulator of neurodegenerative diseases, in particular Alzheimer's disease, said assay comprising:

(a) contacting a cell with a test compound;
(b) measuring the activity and/or level of one or more substances selected from the group consisting of
(i) a gene coding for DAX-1, and/or
(ii) a transcription product of a gene coding for DAX-1, and/or
(iii) a translation product of a gene coding for DAX-1; in said cell
(c) measuring the activity and/or level of one or more substances recited in (i) to (iii) in a control cell not contacted with said test compound; and
(d) comparing the levels and/or activities of the substance in the cells of step (b) and (c), wherein an alteration in the activity and/or level of substances in the contacted cells indicates that the test compound is a modulator of said diseases or disorders.

6. A method of screening for a modulator of neurodegenerative diseases, in particular Alzheimer's disease, said method comprising:

(a) measuring the activity and/or level of one or more substances selected from the group consisting of
(i) a gene coding for DAX-1, and/or
(ii) a transcription product of a gene coding for DAX-1, and/or
(iii) a translation of a gene coding for DAX-1, in a sample of a non-human test animal to which has been administered a test compound wherein non-human test animal is predisposed to developing or has already developed symptoms of a neurodegenerative disease;
(b) measuring the activity and/or level of one or more substances recited in (i) or (iii) in a sample of a matched control animal which is predisposed to developing or has already developed symptoms of a neurodegenerative disease and to which animal no such test compound has been administered;
(c) comparing the activity and/or level of the substance in the animals of step (a) and (b), wherein an alteration in the activity and/or level of substances in the test animal indicates that the test compound is a modulator of said diseases.

7. The method according to claim 6 wherein said test animal and said control animal is a genetically altered non-human animal which expresses the gene coding for DAX-1, under the control of a transcriptional control element which is not the native DAX-1 gene transcriptional control element.

8. The in vitro use of a protein molecule of SEQ ID NO. 1, said protein molecule being a translation product of the gene coding for DAX-1 as diagnostic target for detecting a neurodegenerative disease, preferably Alzheimer's disease.

9. The in vitro use of a protein molecule of SEQ ID NO. 1, said protein molecule being a translation product of the gene coding for DAX-1, as screening target for reagents or compounds preventing, or treating, or ameliorating a neurodegenerative disease, preferably Alzheimer's disease.

10. Use of an antibody specifically immunoreactive with an immunogen, wherein said immunogen is a translation product of a gene coding for DAX-1, SEQ ID NO. 1 for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell which relates to a neurodegenerative disease, preferably to Alzheimer's disease.

**Patentansprüche**

1. Verfahren zum Diagnostizieren oder Prognostizieren einer neurodegenerativen Krankheit bei einem Patienten oder zum Bestimmen, ob ein Patient ein erhöhtes Risiko hat, diese Krankheit zu entwickeln, umfassend das Bestimmen einer Konzentration und/oder einer Aktivität von

(i) einem Transcriptionsprodukt eines Gens, das für DAX-1 codiert; und/oder
(ii) einem Translationsprodukt eines Gens, das für DAX-1 codiert;
in einer Probe, die von dem Patienten erhalten wurde, und Vergleichen der Konzentration und/oder der Aktivität mit einem Referenzwert, der einen bekannten Krankheits- oder Gesundheitszustand darstellt, wodurch die neurodegenerative Krankheit bei dem Patienten diagnostiziert oder prognostiziert wird oder bestimmt wird, ob der Patient ein erhöhtes Risiko hat, diese neurodegenerative Krankheit zu entwickeln.

**2.** Verfahren gemäß Anspruch 1, wobei es sich bei der neurodegenerativen Krankheit um Alzheimer-Krankheit handelt.

**3.** Genetisch verändertes nichthumanes Tier, das die nichtnative Gensequenz von SEQ ID Nr. 3, die für DAX-1 codiert, umfasst, wobei das nichthumane Tier ein Nager, vorzugsweise eine Maus, eine Ratte oder ein Meerschweinchen, oder ein wirbelloses Tier, vorzugsweise ein Insekt, besonders bevorzugt eine Fliege, wie die Fliege *Drosophila melanogaster,* ist, wobei die Expression des Gens dazu führt, dass das nichthumane Tier ein reduziertes Risiko aufweist, Symptome, die denen einer neurodegenerativen Krankheit ähnlich sind, zu entwickeln, insbesondere ein reduziertes Risiko, Symptome, die denen von AD (Alzheimer-Krankheit) ähnlich sind, zu entwickeln, und/oder dass eine Reduktion von AD-Symptomen zeigt und/oder das aufgrund einer Wirkung, die durch die Expression des zur genetischen Veränderung des nichthumanen Tiers verwendeten Gens verursacht wird, keine AD-Symptome aufweist.

**4.** Verwendung des genetisch veränderten nichthumanen Tiers gemäß Anspruch 3 zum Screening, Testen und Validieren von Verbindungen, Mitteln und Modulatoren bei der Entwicklung von Diagnostika und Therapeutika zur Behandlung von neurodegenerativen Krankheiten, insbesondere der Alzheimer-Krankheit.

**5.** In-vitro-Assay zum Screening nach einem Modulator von neurodegenerativen Krankheiten, insbesondere Alzheimer-Krankheit, wobei der Assay Folgendes umfasst:

(a) In-Kontakt-Bringen einer Zelle mit einer Testverbindung;
(b) Messen der Aktivität und/oder der Konzentration von einer oder mehreren Substanzen, die aus der Gruppe ausgewählt sind, die aus

(i) einem Gen, das für DAX-1 codiert, und/oder
(ii) einem Transcriptionsprodukt eines Gens, das für DAX-1 codiert; und/oder
(iii) einem Translationsprodukt eines Gens, das für DAX-1 codiert,
besteht, in der Zelle;

(c) Messen der Aktivität und/oder der Konzentration von einer oder mehreren Substanzen, die in (i) bis (iii) genannt sind, in einer Kontrollzelle, die nicht mit der Testverbindung in Kontakt gebracht wurde; und
(d) Vergleichen der Konzentrationen und/oder Aktivitäten der Substanz in den Zellen der Schritte (b) und (c), wobei eine Veränderung der Aktivität und/oder der Konzentration der Substanzen in den kontaktierten Zellen anzeigt, dass die Testverbindung ein Modulator der Krankheiten oder Störungen ist.

**6.** Verfahren zum Screening nach einem Modulator von neurodegenerativen Krankheiten, insbesondere Alzheimer-Krankheit, wobei das Verfahren Folgendes umfasst:

(a) Messen der Aktivität und/oder der Konzentration von einer oder mehreren Substanzen, die aus der Gruppe ausgewählt sind, die aus

(i) einem Gen, das für DAX-1 codiert, und/oder
(ii) einem Transcriptionsprodukt eines Gens, das für DAX-1 codiert; und/oder
(iii) einem Translationsprodukt eines Gens, das für DAX-1 codiert,
besteht, in einer Probe von einem nichthumanen Versuchstier, dem eine Testverbindung verabreicht wurde, wobei das nichthumane Versuchstier prädisponiert ist, Symptome einer neurodegenerativen Krankheit zu entwickeln, oder bereits welche entwickelt hat;

(b) Messen der Aktivität und/oder der Konzentration von einer oder mehreren Substanzen, die in (i) oder (iii) genannt sind, in einer Probe von einem angepassten Kontrolltier, das prädisponiert ist, Symptome einer neurodegenerativen Krankheit zu entwickeln, oder bereits welche entwickelt hat, wobei dem Kontrolltier keine solche Testverbindung verabreicht wurde;

(c) Vergleichen der Aktivität und/oder der Konzentration der Substanz in den Tieren der Schritte (a) und (b), wobei eine Veränderung der Aktivität und/oder der Konzentration der Substanzen in dem Versuchstier anzeigt, dass die Testverbindung ein Modulator der Krankheiten ist.

**7.** Verfahren gemäß Anspruch 6, wobei das Versuchstier und das Kontrolltier genetisch veränderte nichthumane Tiere sind, die das für DAX-1 codierende Gen unter der Kontrolle eines Transcriptionskontrollelements exprimieren, das nicht das native Transcriptionskontrollelement des DAX-1-Gens ist.

**8.** In-vitro-Verwendung eines Proteinmoleküls von SEQ ID Nr. 1, wobei das Proteinmolekül ein Translationsprodukt des für DAX-1 codierenden Gens ist, als diagnostisches Target zum Nachweisen einer neurodegenerativen Krankheit, vorzugsweise Alzheimer-Krankheit.

**9.** In-vitro-Verwendung eines Proteinmoleküls von SEQ ID Nr. 1, wobei das Proteinmolekül ein Translationsprodukt des für DAX-1 codierenden Gens ist, als Screening-Target für Reagentien oder Verbindungen, die eine neurodegenerative Krankheit, vorzugsweise Alzheimer-Krankheit, verhindern oder behandeln oder lindern.

**10.** Verwendung eines Antikörpers, der gegenüber einem Immunogen spezifisch immunreaktiv ist, wobei das Immunogen ein Translationsprodukt eines für DAX-1 codierenden Gens, SEQ ID Nr. 1, ist, zum Nachweisen des pathologischen Zustands einer Zelle in einer Probe, die von einem Patienten erhalten wurde, umfassend das immuncytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle, der sich auf eine neurodegenerative Krankheit, vorzugsweise Alzheimer-Krankheit, bezieht, anzeigt.

## Revendications

**1.** Procédé pour diagnostiquer ou pronostiquer une maladie neurodégénérative chez un sujet, ou pour déterminer si un sujet présente un risque accru de développer ladite maladie, comprenant la détermination d'un niveau et/ou d'une activité

(i) d'un produit de transcription d'un gène codant pour DAX-1, et/ou
(ii) d'un produit de translation d'un gène codant pour DAX-1
dans un échantillon provenant dudit sujet et la comparaison dudit niveau et/ou de ladite activité à une valeur de référence représentant une maladie ou un état de santé connus, diagnostiquant ou pronostiquant ainsi ladite maladie neurodégénérative chez ledit sujet, ou déterminant si ledit sujet présente un risque accru de développer ladite maladie neurodégénérative.

**2.** Procédé selon la revendication 1, dans lequel ladite maladie neurodégénérative est la maladie d'Alzheimer.

**3.** Animal non humain génétiquement modifié comprenant la séquence du gène non natif de SEQ ID NO. 3 codant pour DAX-1, dans lequel ledit animal non humain est un rongeur, de préférence une souris, un rat ou un cochon d'inde, ou un animal invertébré, de préférence un insecte, de manière davantage préférée une mouche telle que la mouche *Drosophila melanogaster,* dans lequel l'expression dudit gène conduit à ce que ledit animal non humain ait un risque réduit de développer des symptômes similaires à une maladie neurodégénérative, en particulier un risque réduit de développer des symptômes similaires à la maladie d'Alzheimer et/ou qui présente une réduction des symptômes de la maladie d'Alzheimer et/ou qui n'a pas de symptômes de la maladie d'Alzheimer du fait d'un effet provoqué par l'expression du gène utilisé pour modifier génétiquement ledit animal non humain.

**4.** Utilisation de l'animal non humain génétiquement modifié selon la revendication 3 pour le criblage, la mise à l'essai et la validation de composés, agents et modulateurs dans le développement de diagnostiques et de thérapies pour traiter les maladies neurodégénératives, en particulier la maladie d'Alzheimer.

**5.** Dosage *in vitro* pour cribler un modulateur de maladies neurodégénératives, en particulier la maladie d'Alzheimer, ledit dosage comprenant :

(a) la mise en contact d'une cellule avec un composé d'essai ;
(b) la mesure de l'activité et/ou du niveau d'une ou plusieurs substances choisies dans le groupe consistant en

(i) un gène codant pour DAX-1, et/ou
(ii) un produit de transcription d'un gène codant pour DAX-1, et/ou
(iii) un produit de translation d'un gène codant pour DAX-1, dans ladite cellule

(c) la mesure de l'activité et/ou du niveau d'une ou plusieurs substances évoquées dans (i) à (iii) dans une cellule témoin qui n'est pas mise en contact avec ledit composé d'essai ; et
(d) la comparaison des niveaux et/ou activités de la substance dans les cellules des étapes (b) et (c), dans lequel une modification de l'activité et/ou du niveau de substances dans les cellules mises en contact indique que le composé d'essai est un modulateur desdites maladies ou troubles.

**6.** Procédé de criblage pour un modulateur de maladies neurodégénératives, en particulier la maladie d'Alzheimer, ledit procédé comprenant :

(a) la mesure de l'activité et/ou du niveau d'une ou plusieurs substances choisies dans le groupe consistant en

(i) un gène codant pour DAX-1, et/ou
(ii) un produit de transcription d'un gène codant pour DAX-1, et/ou
(iii) un produit de translation d'un gène codant pour DAX-1, dans un échantillon d'un animal d'essai non humain auquel un composé d'essai a été administré, dans lequel l'animal d'essai non humain est prédisposé à développer ou a déjà développé des symptômes d'une maladie neurodégénérative ;

(b) la mesure de l'activité et/ou du niveau d'une ou plusieurs substances évoquées dans (i) ou (iii) dans un échantillon d'animal témoin apparié qui est prédisposé à développer ou a déjà développé des symptômes d'une maladie neurodégénérative et auquel animal aucun composé d'essai de ce type n'a été administré ;
(c) la comparaison de l'activité et/ou du niveau de la substance chez les animaux des étapes (a) et (b), dans lequel une modification de l'activité et/ou du niveau de substances chez l'animal d'essai indique que le composé d'essai est un modulateur desdites maladies.

**7.** Procédé selon la revendication 6, dans lequel ledit animal d'essai et ledit animal témoin sont un animal non humain génétiquement modifié qui exprime le gène codant pour DAX-1, sous le contrôle d'un élément de contrôle transcriptionnel qui n'est pas l'élément de contrôle transcriptionnel du gène DAX-1 natif.

**8.** Utilisation *in vitro* d'une molécule protéique de SEQ ID NO. 1, ladite molécule protéique étant un produit de translation du gène codant pour DAX-1, comme cible de diagnostic pour détecter une maladie neurodégénérative, de préférence la maladie d'Alzheimer.

**9.** Utilisation *in vitro* d'une molécule protéique de SEQ ID NO. 1, ladite molécule protéique étant un produit de translation du gène codant pour DAX-1, comme cible de criblage pour des réactifs ou des composés prévenant, ou traitant ou améliorant une maladie neurodégénérative, de préférence la maladie d'Alzheimer.

**10.** Utilisation d'un anticorps spécifiquement immunoréactif avec un immunogène, dans laquelle ledit immunogène est un produit de translation d'un gène codant pour DAX-1, SEO ID NO. 1 pour détecter l'état pathologique d'une cellule dans un échantillon provenant d'un sujet, comprenant la coloration immunocytochimique de ladite cellule avec ledit anticorps, dans laquelle un degré modifié de coloration, ou un motif de coloration modifié dans ladite cellule comparé à une cellule représentant un état de santé connu indique un état pathologique de ladite cellule qui se rapporte à une maladie neurodégénérative, de préférence à la maladie d'Alzheimer.

# Figure 1: Verification of differential expression of human DAX-1 by quantitative RT-PCR

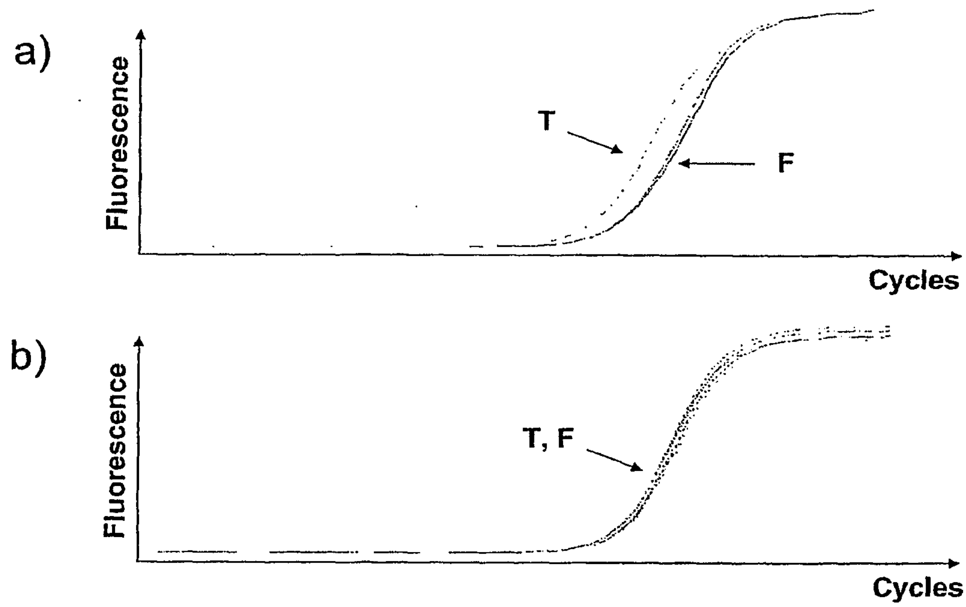

# Figure 2: Verification of differential expression of human DAX-1 by quantitative RT-PCR

a)

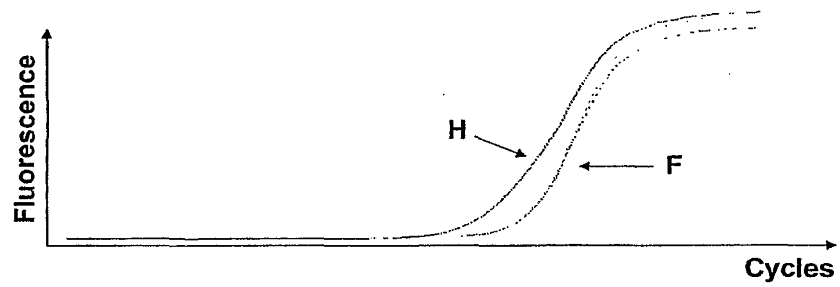


b)

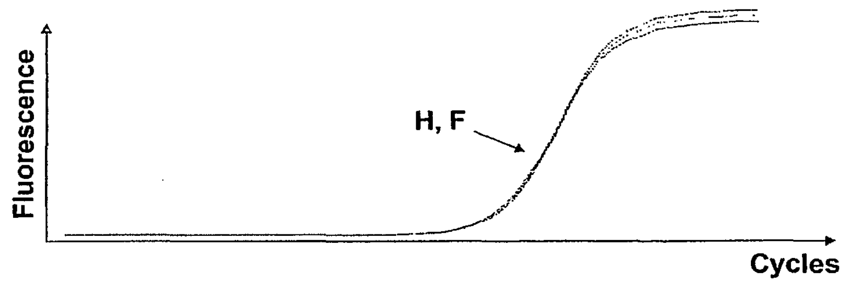

# Fig. 3: Analysis of absolute mRNA expression of DAX-1

Comparison of Braak 0-3 with 4-6
ens 1054

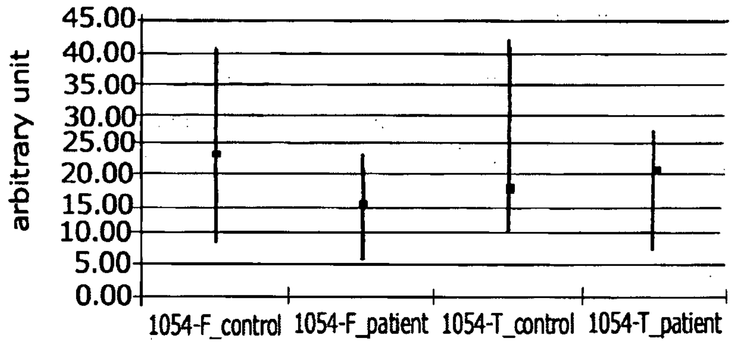


Comparison of Braak 0-2 with 3-6
ens 1054

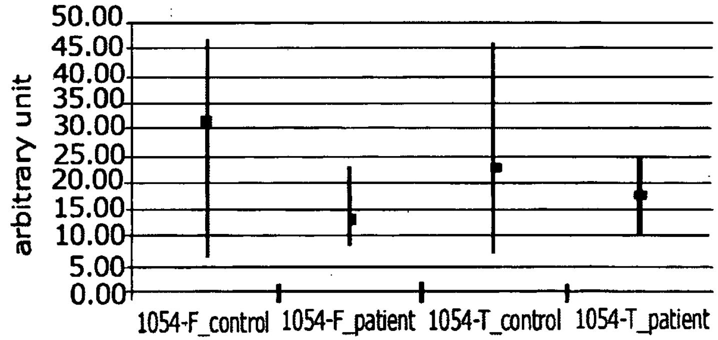


Comparison of Braak 0-1 with 2-6
ens 1054

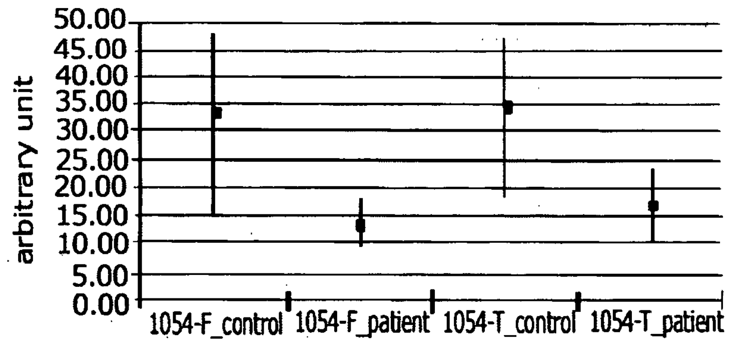


Comparison of Braak 0-1 with 2-3 and 4-6
ens 1054

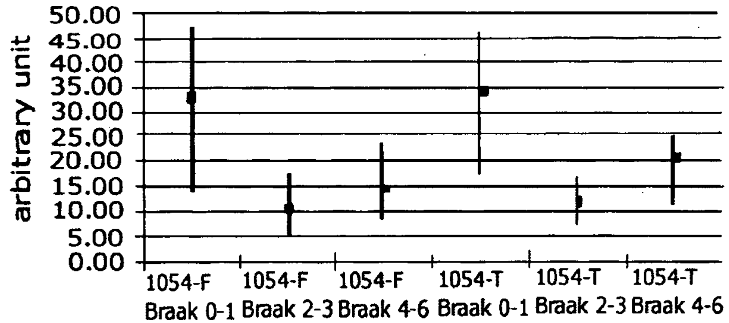

# Figure 4: SEQ ID NO. 1: amino acid sequence of human DAX-1 protein

**Length: 470 aa**

```
  1  MAGENHQWQG SILYNMLMSA KQTRAAPEAP ETRLVDQCWG CSCGDEPGVG
 51  REGLLGGRNV ALLYRCCFCG KDHPRQGSIL YSMLTSAKQT YAAPKAPEAT
101  LGPCWGCSCG SDPGVGRAGL PGGRPVALLY RCCFCGEDHP RQGSILYSLL
151  TSSKQTHVAP AAPEARPGGA WWDRSYFAQR PGGKEALPGG RATALLYRCC
201  FCGEDHPQQG STLYCVPTST NQAQAAPEER PRAPWWDTSS GALRPVALKS
251  PQVVCEAASA GLLKTLRFVK YLPCFQVLPL DQQLVLVRNC WASLLMLELA
301  QDRLQFETVE VSEPSMLQKI LTTRRRETGG NEPLPVPTLQ HHLAPPAEAR
351  KVPSASQVQA IKCFLSKCWS LNISTKEYAY LKGTVLFNPD VPGLQCVKYI
401  QGLQWGTQQI LSEHTRMTHQ GPHDRFIELN STLFLLRFIN ANVIAELFFR
451  PIIGTVSMDD MMLEMLCTKI
```

# Figure 5: SEQ ID NO. 2: human DAX-1 cDNA nucleotide sequence

**Length: 2022 bp**

```
   1  GAGCTCCCAC GCTGCTGTTC TTCCATTTCC AGCTTTTAAA GAGCACCCGC
  51  CCCTTCGAAC CACCGAGGTC ATGGGCGAAC ACACCGGAGC GCAGACCGCG
 101  CCCCCCCGCA CACACCGCCC GCCTCCGCGC CCTTGCCCAG ACCGAGGCGG
 151  CCGACGCGCC TGCGTGCGCG CTAGGTATAA ATAGGTCCCA GGAGGCAGCC
 201  ACTGGGCAGA ACTGGGCTAC GGGCGCCGCG GGCCATGGCG GGCGAGAACC
 251  ACCAGTGGCA GGGCAGCATC CTCTACAACA TGCTTATGAG CGCGAAGCAA
 301  ACGCGCGCGG CTCCTGAGGC TCCAGAGACG CGGCTGGTGG ATCAGTGTTG
 351  GGGCTGTTCG TGCGGCGATG AGCCCGGGGT GGGCAGAGAG GGGCTGCTGG
 401  GCGGGCGGAA CGTGGCGCTC CTGTACCGCT GCTGCTTTTG CGGTAAAGAC
 451  CACCCACGGC AGGGCAGCAT CCTCTACAGC ATGCTGACGA GCGCAAAGCA
 501  AACGTACGCG GCACCGAAGG CGCCCGAGGC GACGCTGGGT CCGTGCTGGG
 551  GCTGTTCGTG CGGCTCTGAT CCCGGGGTGG GCAGAGCGGG GCTTCCGGGT
 601  GGGCGGCCCG TGGCACTCCT GTACCGCTGC TGCTTTTGTG GTGAAGACCA
 651  CCCGCGGCAG GGCAGCATCC TCTACAGCTT GCTCACTAGC TCAAAGCAAA
 701  CGCACGTGGC TCCGGCAGCG CCCGAGGCAC GGCCAGGGGG CGCGTGGTGG
 751  GACCGCTCCT ACTTCGCGCA GAGGCCAGGG GGTAAAGAGG CGCTACCAGG
 801  CGGGCGGGCC ACGGCGCTTC TGTACCGCTG CTGCTTTTGC GGTGAAGACC
 851  ACCCGCAGCA GGGCAGCACC CTCTACTGCG TGCCCACGAG CACAAATCAA
 901  GCGCAGGCGG CTCCGGAGGA GCGGCCGAGG GCCCCCTGGT GGGACACCTC
 951  CTCTGGTGCG CTGCGGCCGG TGGCGCTCAA GAGTCCACAG GTGGTCTGCG
1001  AGGCAGCCTC AGCGGGCCTG TTGAAGACGC TGCGCTTCGT CAAGTACTTG
1051  CCCTGCTTCC AGGTGCTGCC CCTGGACCAG CAGCTGGTGC TGGTGCGCAA
1101  CTGCTGGGCG TCCCTGCTCA TGCTTGAGCT GGCCCAGGAC CGCTTGCAGT
1151  TCGAGACTGT GGAAGTCTCG GAGCCCAGCA TGCTGCAGAA GATCCTCACC
1201  ACCAGGCGGC GGGAGACCGG GGGCAACGAG CCACTGCCCG TGCCCACGCT
1251  GCAGCACCAT TTGGCACCGC CGGCGGAGGC CAGGAAGGTG CCCTCCGCCT
1301  CCCAGGTCCA AGCCATCAAG TGCTTTCTTT CCAAATGCTG GAGTCTGAAC
1351  ATCAGTACCA AGGAGTACGC CTACCTCAAG GGGACCGTGC TCTTTAACCC
1401  GGACGTGCCG GGCCTGCAGT GCGTGAAGTA CATTCAGGGA CTCCAGTGGG
1451  GAACTCAGCA AATACTCAGT GAACACACCA GGATGACGCA CCAAGGGCCC
1501  CATGACAGAT TCATCGAACT TAATAGTACC CTTTTCCTGC TGAGATTCAT
1551  CAATGCCAAT GTCATTGCTG AACTGTTCTT CAGGCCCATC ATCGGCACAG
1601  TCAGCATGGA TGATATGATG CTGGAAATGC TCTGTACAAA GATATAAAGT
1651  CATGTGGGCC ACACAAGTGC AGTAGTGCAG TTCACCATGA GGGAAGAATA
1701  AAGAGCTGTG GGCAAAAGAG TGTAAAATAT TTTAAAATAA ACTTTCTTAA
1751  TATTTTTACA TGCAGAGTAT TTTGATCTTC AATTAAAGAA ATAATTTTAT
1801  TCCCAGCACA GTCACAAATT TCTCTGTTCC ATAGTTAAAG AAGACATTTG
1851  CCAACAGGTA GCATAGCTCT GTACATCTTT TAAAAAAAAA ATCGCAGGGT
1901  ACTAGTATAA TAAGCTATTT TCACAAGCGC AGCAATTTCA TGGAACCTGC
1951  TCAAATCAAA TTTGTACATA TTGTTATAAT AAATTTTAAG GTCTTAACTA
2001  TTAACTTGAT TGAAAAAAGC TT
```

# Figure 6: SEQ ID NO. 3: nucleotide sequence of human DAX-1 coding sequence

**Length: 1413 bp**

```
   1 ATGGCGGGCG AGAACCACCA GTGGCAGGGC AGCATCCTCT ACAACATGCT
  51 TATGAGCGCG AAGCAAACGC GCGCGGCTCC TGAGGCTCCA GAGACGCGGC
 101 TGGTGGATCA GTGTTGGGGC TGTTCGTGCG GCGATGAGCC CGGGGTGGGC
 151 AGAGAGGGGC TGCTGGGCGG GCGGAACGTG GCGCTCCTGT ACCGCTGCTG
 201 CTTTTGCGGT AAAGACCACC CACGGCAGGG CAGCATCCTC TACAGCATGC
 251 TGACGAGCGC AAAGCAAACG TACGCGGCAC CGAAGGCGCC CGAGGCGACG
 301 CTGGGTCCGT GCTGGGGCTG TTCGTGCGGC TCTGATCCCG GGGTGGGCAG
 351 AGCGGGGCTT CCGGGTGGGC GGCCCGTGGC ACTCCTGTAC CGCTGCTGCT
 401 TTTGTGGTGA AGACCACCCG CGGCAGGGCA GCATCCTCTA CAGCTTGCTC
 451 ACTAGCTCAA AGCAAACGCA CGTGGCTCCG GCAGCGCCCG AGGCACGGCC
 501 AGGGGGCGCG TGGTGGGACC GCTCCTACTT CGCGCAGAGG CCAGGGGGTA
 551 AAGAGGCGCT ACCAGGCGGG CGGGCCACGG CGCTTCTGTA CCGCTGCTGC
 601 TTTTGCGGTG AAGACCACCC GCAGCAGGGC AGCACCCTCT ACTGCGTGCC
 651 CACGAGCACA AATCAAGCGC AGGCGGCTCC GGAGGAGCGG CCGAGGGCCC
 701 CCTGGTGGGA CACCTCCTCT GGTGCGCTGC GGCCGGTGGC GCTCAAGAGT
 751 CCACAGGTGG TCTGCGAGGC AGCCTCAGCG GGCCTGTTGA AGACGCTGCG
 801 CTTCGTCAAG TACTTGCCCT GCTTCCAGGT GCTGCCCCTG GACCAGCAGC
 851 TGGTGCTGGT GCGCAACTGC TGGGCGTCCC TGCTCATGCT TGAGCTGGCC
 901 CAGGACCGCT TGCAGTTCGA GACTGTGGAA GTCTCGGAGC CCAGCATGCT
 951 GCAGAAGATC CTCACCACCA GGCGGCGGGA GACCGGGGGC AACGAGCCAC
1001 TGCCCGTGCC CACGCTGCAG CACCATTTGG CACCGCCGGC GGAGGCCAGG
1051 AAGGTGCCCT CCGCCTCCCA GGTCCAAGCC ATCAAGTGCT TTCTTTCCAA
1101 ATGCTGGAGT CTGAACATCA GTACCAAGGA GTACGCCTAC CTCAAGGGGA
1151 CCGTGCTCTT TAACCCGGAC GTGCCGGGCC TGCAGTGCGT GAAGTACATT
1201 CAGGGACTCC AGTGGGGAAC TCAGCAAATA CTCAGTGAAC ACACCAGGAT
1251 GACGCACCAA GGGCCCCATG ACAGATTCAT CGAACTTAAT AGTACCCTTT
1301 TCCTGCTGAG ATTCATCAAT GCCAATGTCA TTGCTGAACT GTTCTTCAGG
1351 CCCATCATCG GCACAGTCAG CATGGATGAT ATGATGCTGG AAATGCTCTG
1401 TACAAAGATA TAA
```

# Figure 7: Alignment of DAX-1 primers with human DAX-1 cDNA, SEQ ID NO. 2

```
   1 TACCAAGGAGTACGCCTACCTCA 23
     |||||||||||||||||||||||
1356 TACCAAGGAGTACGCCTACCTCA 1378


  20 TGCTCTTTAACCCGGACGTG 1
     ||||||||||||||||||||
1388 TGCTCTTTAACCCGGACGTG 1407
```

Figure 8 :

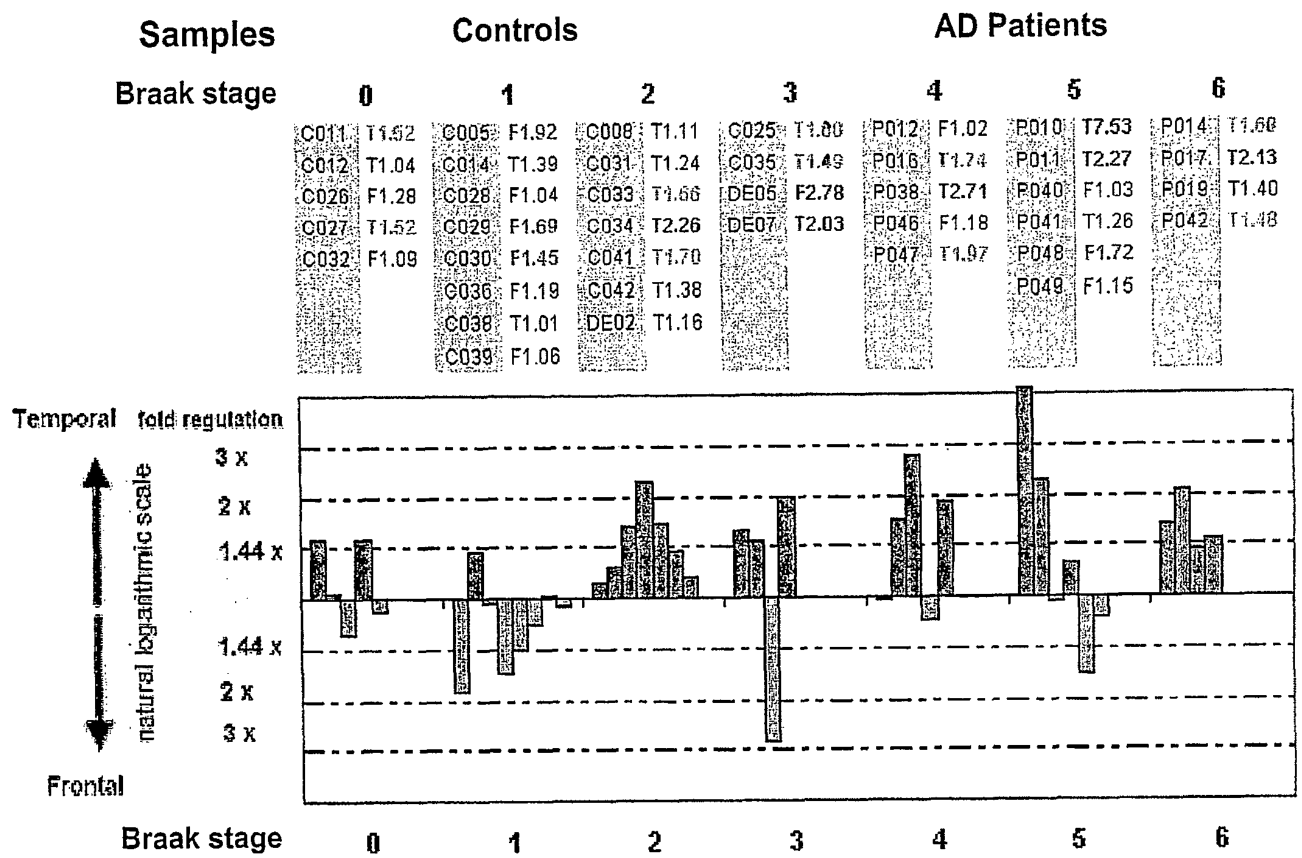
Samples
Controls
AD Patients
Braak stage
0
1
2
3
4
5
6
C011 T1.52
C012 T1.04
C026 F1.28
C027 T1.52
C032 F1.09
C005 F1.92
C014 T1.39
C028 F1.04
C029 F1.69
C030 F1.45
C036 F1.19
C038 T1.01
C039 F1.06
C008 T1.11
C031 T1.24
C033 T1.66
C034 T2.26
C041 T1.70
C042 T1.38
DE02 T1.16
C025 T1.80
C035 T1.49
DE05 F2.78
DE07 T2.03
P012 F1.02
P016 T1.74
P038 T2.71
P046 F1.18
P047 T1.97
P010 T7.53
P011 T2.27
P040 F1.03
P041 T1.26
P048 F1.72
P049 F1.15
P014 T1.60
P017 T2.13
P019 T1.40
P042 T1.48
Temporal
fold regulation
3 x
2 x
1.44 x
1.44 x
2 x
3 x
natural logarithmic scale
Frontal
Braak stage
0
1
2
3
4
5
6

# Figure 9 :

| sample | Δ (fold) (hippocampus/ frontal cortex) |
|---|---|
| control C005 | 0.08 |
| control C008 | 2.23 |
| control C004 | 1.93 |
| patient P012 | 1.27 |
| patient P016 | 4.11 |
| patient P010 | 13.61 |
| patient P011 | 1.14 |
| patient P014 | 2.28 |
| patient P019 | 2.77 |

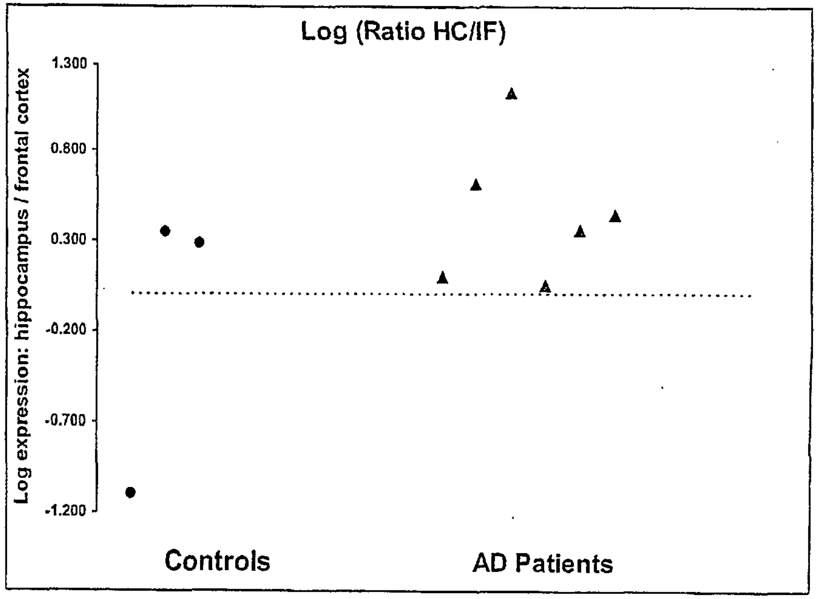

Figure 10: Western Blot of total human brain extracts labeled with anti-DAX-1 antibodies

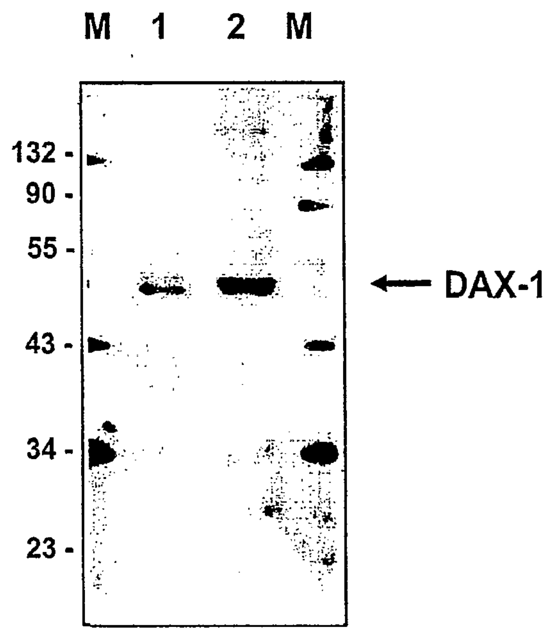

Figure 11: Images of human brain sections labeled with anti-DAX-1 antiserum and with DAPI

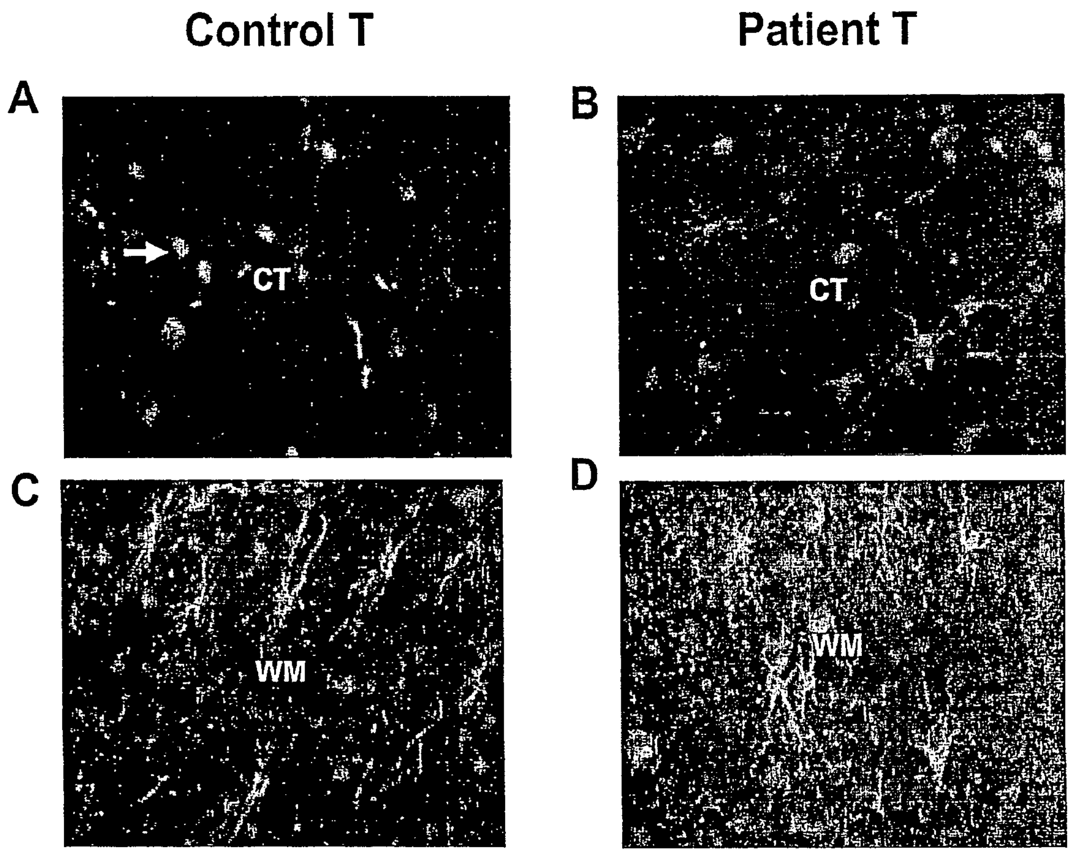

# Figure 12: Immunofluorescence analysis of DAX-1 protein in neuroglioma cells

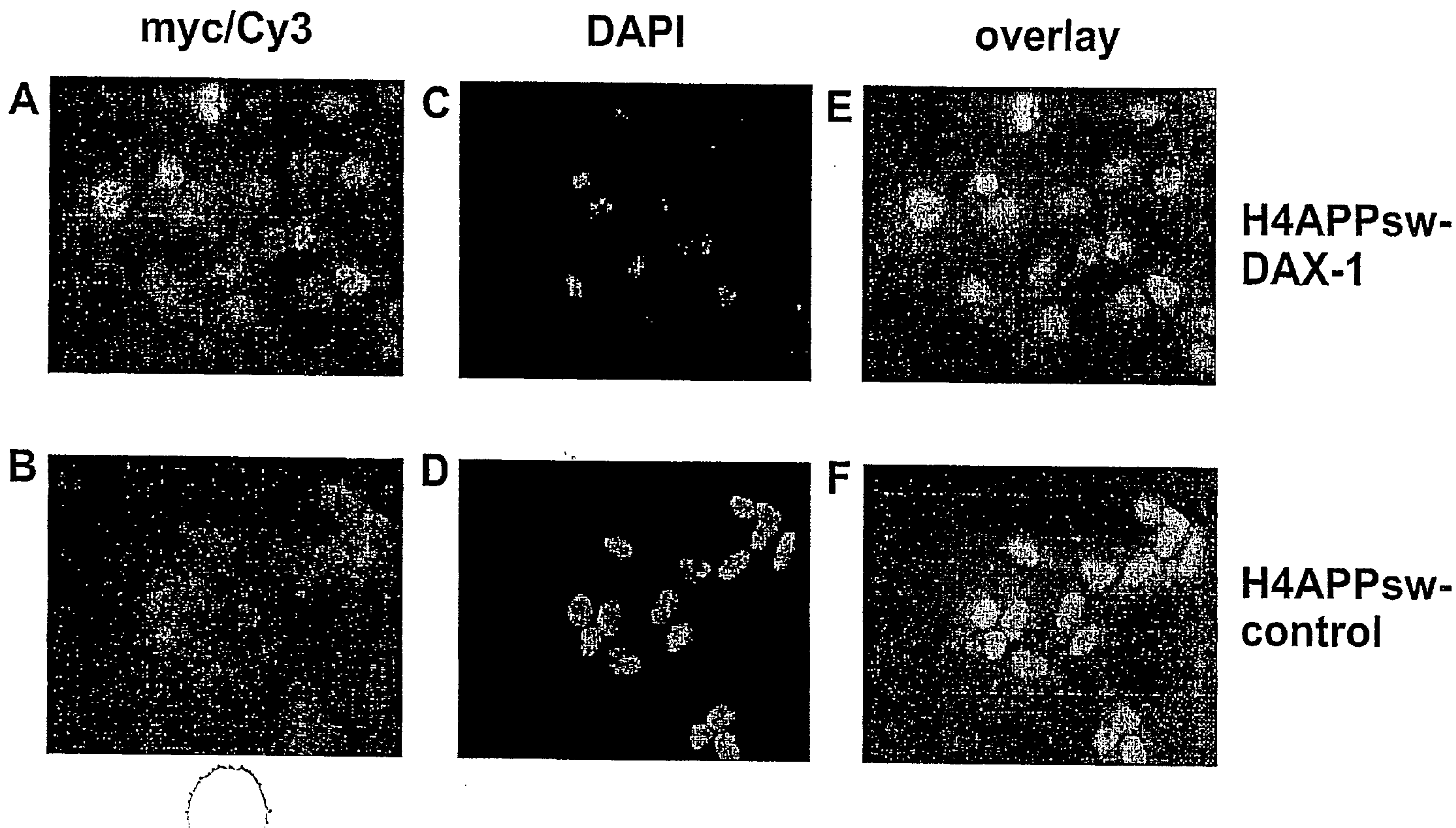

# Figure 13: Effect of trophic factor deprivation on DAX-1 over-expressing cells

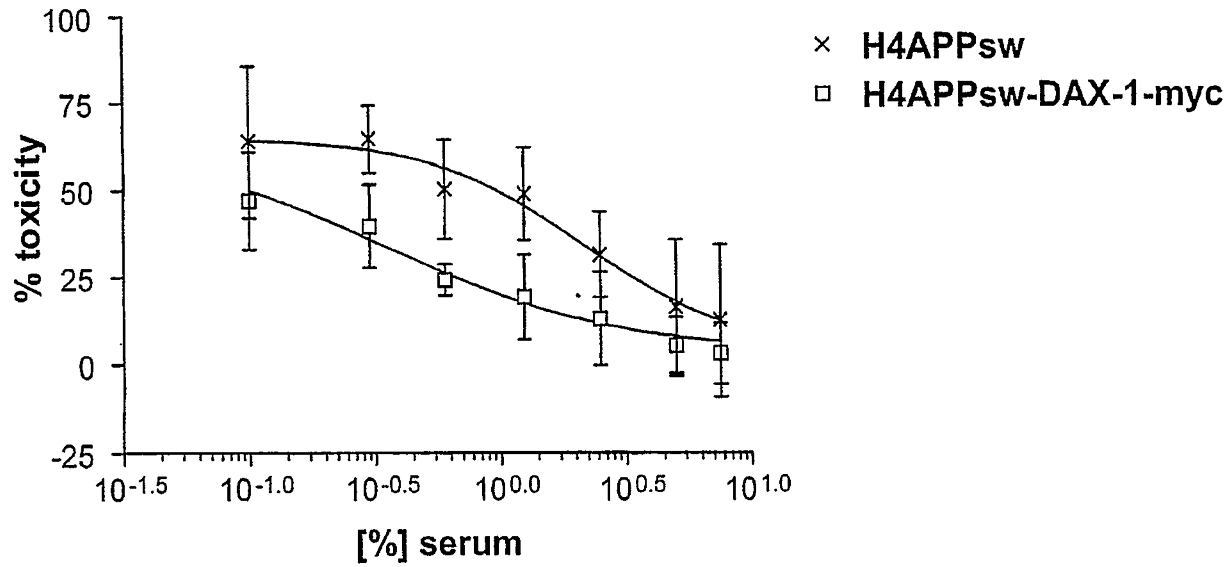

# Figure 14: DAX-1 Protein expression in transgenic flies

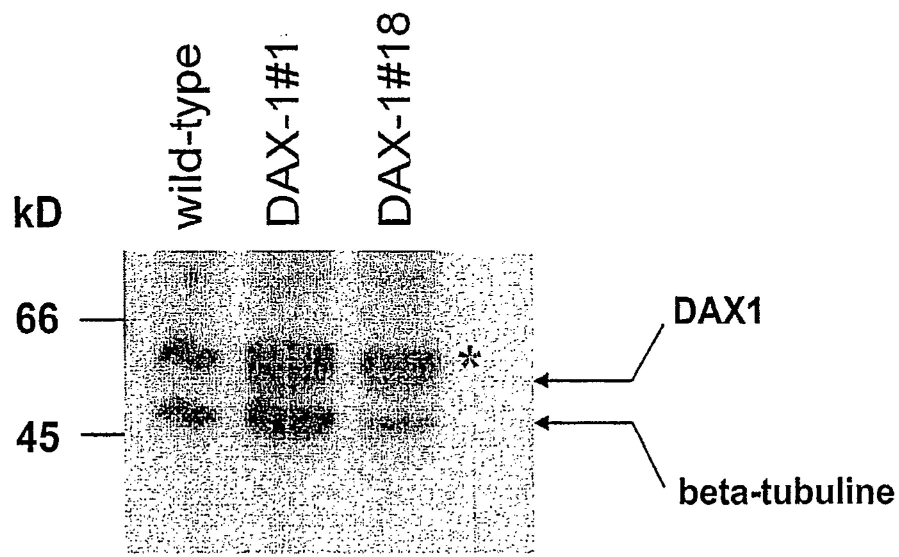

# Figure 15: DAX-1 Protein expression in the retina of adult flies

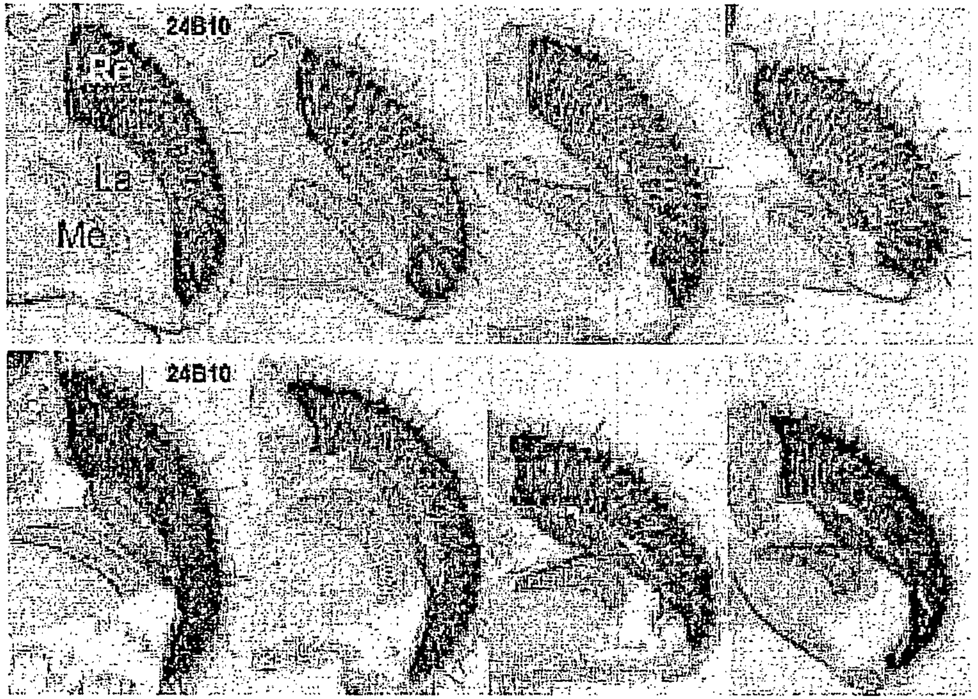

# Figure 16: DAX-1 rescues photoreceptor cell degeneration induced by APP/BACE

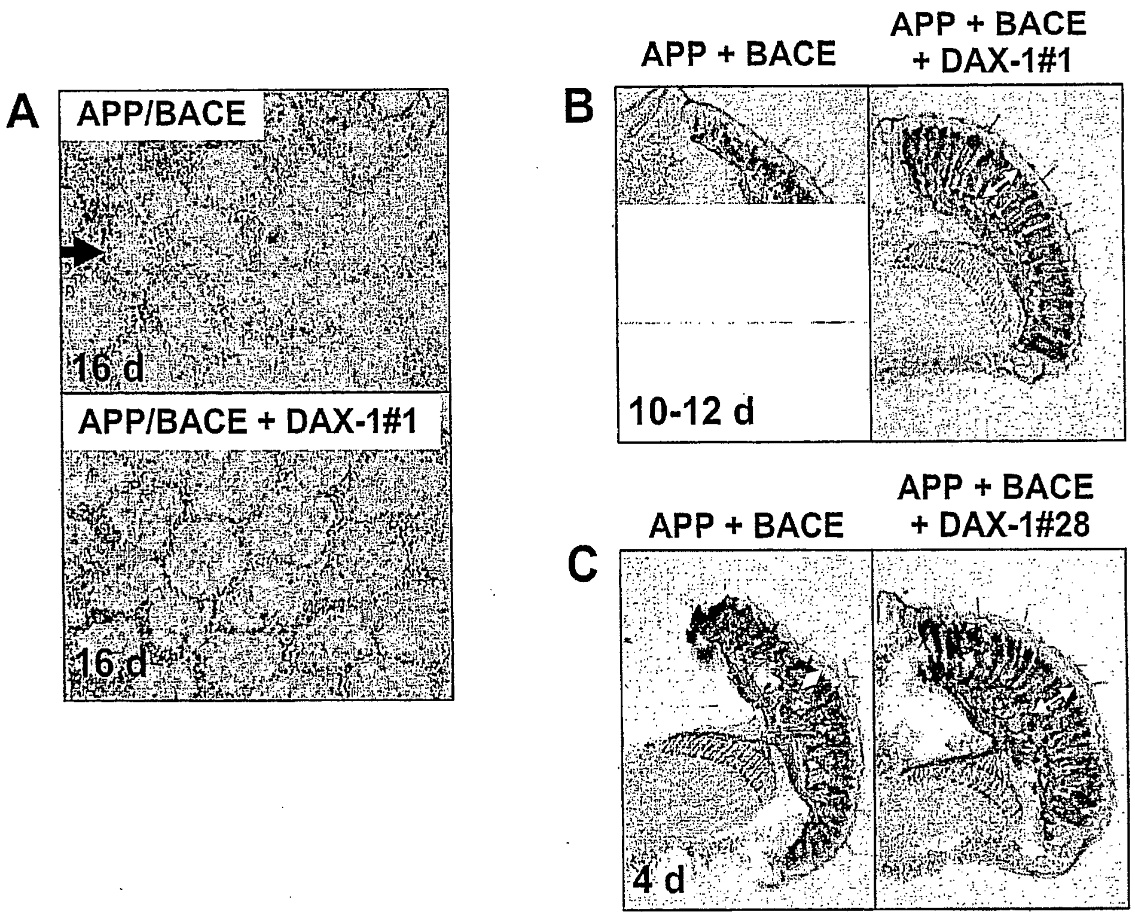

# Figure 17: Thioflavin S positive amyloid plaques in DAX-1 expressing flies

A

APP + BACE + PsnL235P

B

APP + BACE + PsnL235P + DAX-1#1

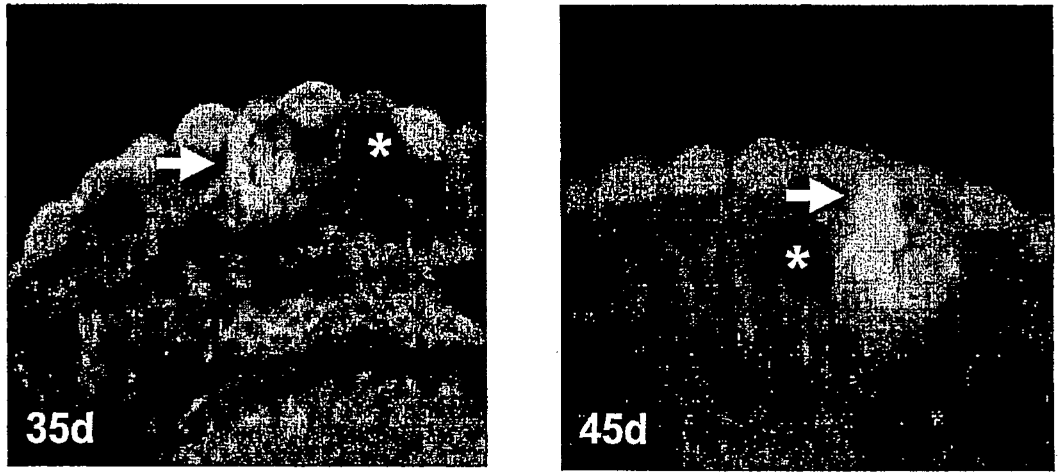

# Figure 18: DAX-1 rescues photoreceptor cell degeneration induced by TAU

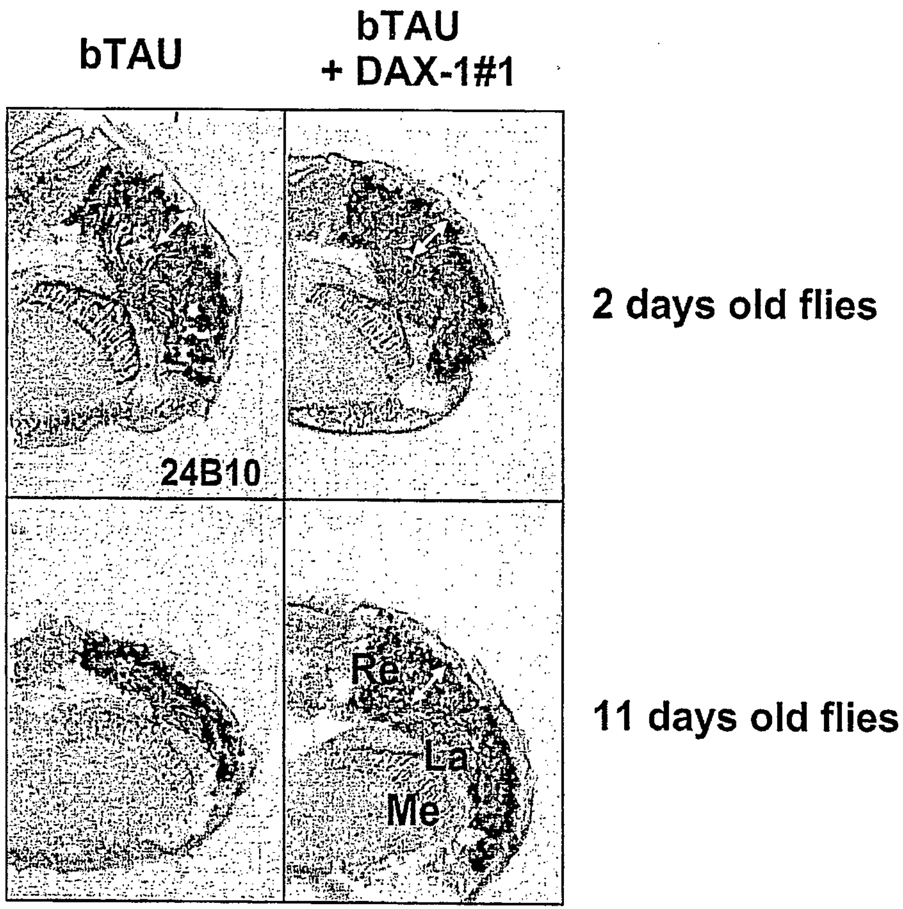

Figure 19: Generation of DAX-1 transgenic mice

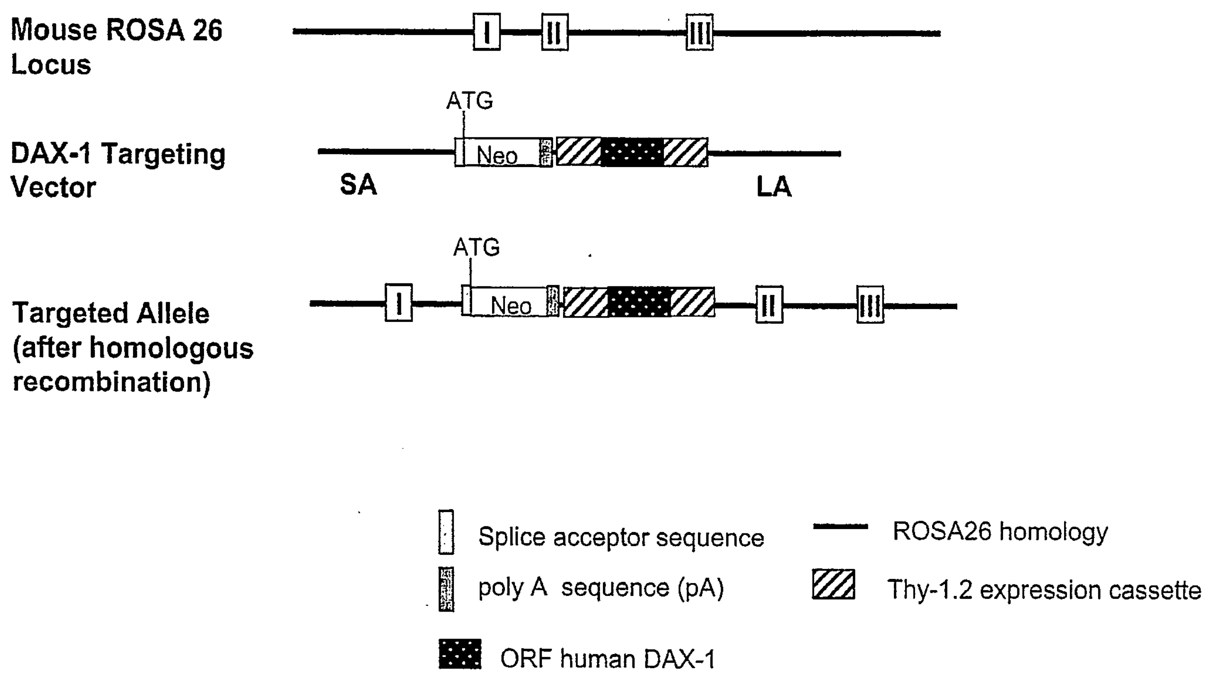

# Figure 20: DAX-1 targeted ES cell clones

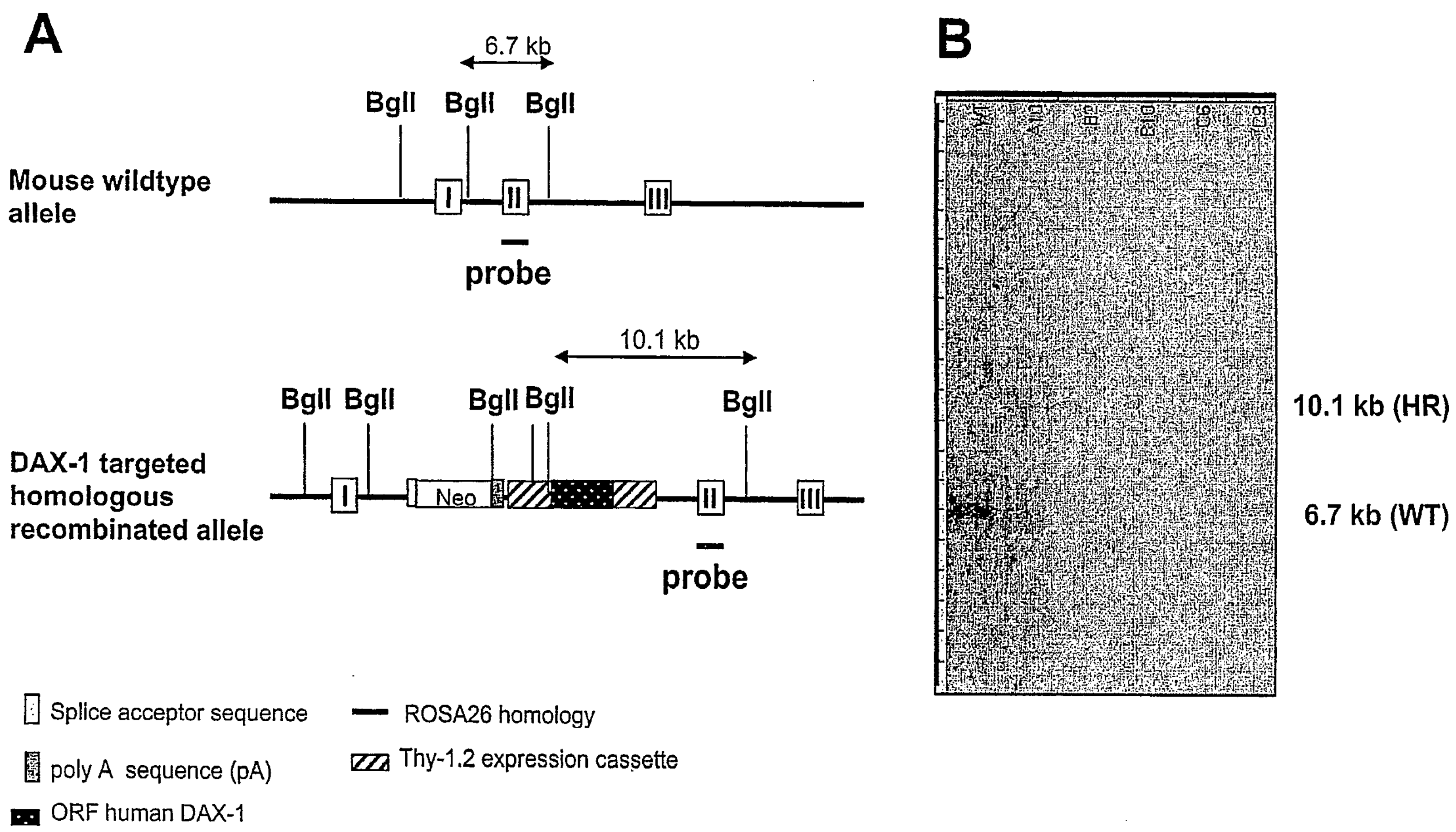

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020068815 A1
- US 6465627 B, McCabe
- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A
- DE 07036226


### Non-patent literature cited in the description


- **VICKERS et al.** *Progress in Neurobiology,* 2000, vol. 60, 139-165
- **SELKOE.** *Physiological Rev,* 2001, vol. 81, 741-66
- **GREENFIELD et al.** *Frontiers Bioscience,* 2000, vol. 5, D72-83
- **BRAAK ; BRAAK.** *Acta Neuropathol,* 1991, vol. 82, 239-259
- **SCHMITT et al.** *Neurology,* 2000, vol. 55, 370-376
- **TERRY et al.** Annals of Neurology. 1981, vol. 10, 184-92
- **CORDER et al.** *Science,* 1993, vol. 261, 921-923
- **STRITTMATTER et al.** *Proc Natl Acad Sci USA,* 1993, vol. 90, 1977-81
- **ROSES.** *Ann NY Acad Sci,* 1998, vol. 855, 738-43
- **HOWLAND et al.** *J. Biol. Chem,* 1998, vol. 273, 16576-16582
- **SIMONS et al.** *Proc. Natl. Acad. Sci. USA,* 1998, vol. 95, 6460-6464
- **JICK et al.** *Lancet,* 2000, vol. 356, 1627-1631
- **WOLOZIN et al.** *Arch. Neurol.,* 2000, vol. 57, 1439-1443
- **KALMIJN et al.** *Ann. Neurol.,* 1997, vol. 42, 776-782
- **ZANARIA et al.** *Nature,* 1994, vol. 372, 635-641
- **GUO et al.** *J. Endocrinol. Met.,* 1996, vol. 81, 2481-2486
- **BURRIS et al.** *Biochem. Biophys. Res. Comm.,* 1995, vol. 214, 576-581
- **GUO et al.** *J. Clin. Endocrinol. Met.,* 1996, vol. 81, 2481-2486
- **PATEL et al.** *J. Invest. Dermatol.,* 2001, vol. 117, 1559-1565
- **GUO et al.** *Biochem. Mol. Med.,* 1995, vol. 56, 8-13
- **LALLI et al.** *Mol. Cell Biol.,* 2000, vol. 20, 4910-4921
- **LEHMANN et al.** *Proc. Natl. Acad. Sci. USA,* 2002, vol. 99, 8225-8230
- **ZAZOPOULOS et al.** *Nature,* 1997, vol. 390, 311-315
- **OSMAN et al.** *J. Biol. Chem.,* 2002, vol. 277, 41259-41267
- **IKEDA et al.** *Dev. Dyn.,* 2001, vol. 220, 363-376
- **LALLI et al.** *Mol. Endocrinol.,* 2003, vol. 17, 1445-1453
- **LALLI et al.** *Mol. Endocrinol.,* 1997, vol. 11, 1950-1960
- **LALLI et al.** *Endocrinol.,* 1998, vol. 139, 4237-4243
- **HANLEY et al.** *Mol. Endocrinol.,* 2001, vol. 15, 57-68
- **WANG et al.** *Proc. Nat. Acad. Sci. USA,* 2001, vol. 98, 7988-7993
- **IKEDA et al.** *Mol. Endocrinol.,* 1996, vol. 10, 1261-1272
- *European Journal of Endocrinology,* 1999, vol. 140, 291-292
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **DEVEREUX et al.** *Nucleic Acids Res.,* 1984, vol. 12, 387
- **PEARSON ; LIPMAN.** *Proc. Natl. Acad. Sci. USA,* 1988, vol. 85, 2444-2448
- **WILBUR ; LIPMAN.** *SIAM J. Appl. Math.,* 1984, vol. 44, 557-567

- **NEEDLEMAN ; WUNSCH.** *J. Mol. Biol.,* 1970, vol. 48, 443-453
- Alzheimer's Disease and Related Disorders. **IQBAL ; SWAAB ; WINBLAD ; WISNIEWSKI.** Etiology, Pathogenesis and Therapeutics. Wiley & Sons, 1999
- **SCINTO ; DAFFNER.** Early Diagnosis of Alzheimer's Disease. Humana Press, 2000
- **MAYEUX ; CHRISTEN.** Epidemiology of Alzheimer's Disease: From Gene to Prevention. Springer Press, 1999
- **YOUNKIN ; TANZI ; CHRISTEN.** Presenilins and Alzheimer's Disease. Springer Press, 1998
- **BRAAK ; BRAAK.** *Acta Neuropathology,* 1991, vol. 82, 239-259
- **TERRY et al.** Annals of Neurology. 1981, vol. 10, 184-192
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **BEHR.** *Acc Chem Res,* 1993, vol. 26, 274-278
- **MULLIGAN.** *Science,* 1993, vol. 260, 926-931
- **WOLFF.** *Curr Opin Neurobiol,* 1993, vol. 3, 743-748
- **GILLESPIE.** *DN&P,* 1992, vol. 5, 389-395
- **AGRAWAL ; AKHTAR.** *Trends Biotechnol,* 1995, vol. 13, 197-199
- **CROOKE.** *Biotechnology,* 1992, vol. 10, 882-6
- **BARINAGA.** *Science,* 1993, vol. 262, 1512-1514
- **WICKSTROM.** *Trends Biotechnol,* 1992, vol. 10, 281-287
- **HANNON.** *Nature,* 2002, vol. 418, 244-251
- **MC CELLAND ; PARDEE.** *Expression Genetics: Accelerated and High-Throughput Methods,* 1999
- **JANUS ; WESTAWAY.** *Physiology Behavior,* 2001, 873-886
- **RICHARDS et al.** *J. Neuroscience,* 2003, vol. 23, 8989-9003
- **GÖTZ et al.** *J. Biological Chemistry,* 2001, vol. 276, 529-534
- **CAPECCHI.** *Science,* 1989, vol. 244, 1288-1292
- **HOGAN et al.** Manipulating the Mouse Embryo: A Laboratory Manual. Cold Spring Harbor Laboratory Press, 1994
- **JACKSON ; ABBOTT.** Mouse Genetics and Transgenics: A Practical Approach. Oxford University Press, 1999
- **SCHWARZ et al.** *Biochemistry,* 1999, vol. 38, 9456-9464
- **KRIVOSHEEV ; USANOV.** *Biochemistry-Moscow,* 1997, vol. 62, 1064-1073
- **HARLOW et al.** Antibodies, A Laboratory Manual. Cold Spring Harbor Laboratory Press, 1988
- **DUBEL ; BREITLING.** Recombinant Antibodies. Wiley-Liss, 1999
- **EDWARDS R.** Immunodiagnostics: A Practical Approach. Oxford University Press
- **GREEVE et al.** *J. Neurosci.,* 2004, vol. 24, 3899-3906
- **BRAND ; PERRIMON.** *Development,* 1993, vol. 118, 401-15
- **RUBIN ; SPRADLING.** *Science,* 1982, vol. 218, 348-53
- **SPRADLING ; RUBIN.** *Science,* 1982, vol. 218, 341-7
- **FOSSGREEN et al.** *Proc Natl Acad Sci U S A,* 1998, vol. 95, 13703-8
- **YE ; FORTINI.** *J Cell Biol,* 1999, vol. 146, 1351-64
- **ITO et al.** *Cell Tissue Res.,* 1997, vol. 290, 1-10
- **TSCHÄPE J.-A. et al.** *EMBO J.,* 2002, vol. 21, 6367-6376
- **IDA et al.** *J Biol Chem,* 1996, vol. 271, 22908-14
- **LUTHI ; VAN DER PUTTEN.** *J. Neuroscience,* 1997, vol. 17, 4688-4699
- **SEIBLER et al.** *Nucleic Acids Research,* 2003, vol. 31, e12
- **FRIEDRICH ; SORIANO.** *Genes Dev.,* 1991, vol. 5, 1513-1523
- **ZAMBROWICZ et al.** *Proc Natl Acad Sci USA,* vol. 94, 3789-3794
- **TYMMS ; KOLA.** Gene Knockout Protocols. Humana Press, 2001